# EUROPEAN PATENT APPLICATION

(11) **EP 3 971 158 A1**
(43) Date of publication of application: **23.03.2022**
(21) Application number: 20809416.9
(22) Date of filing: 15.05.2020
(51) Int. Cl.: C07C 37/52, C07C 37/11, C07C 39/23, C07C 67/343, C07C 69/94, C07C 51/353, C07C 65/19

(54) **METHOD FOR PREPARING CANNABIDIOL COMPOUND**

(30) Priority: 17.05.2019 CN 201910410760; 27.11.2019 CN 201911185126
(71) Applicant: Topharman Shanghai Co., Ltd., Pudong, Shanghai 201203 (CN); Topharman Tancheng Co., Ltd., Linyi, Shandong 276100 (CN); Shanghai Institute of Materia Medica, Chinese Academy of Sciences, Pudong, Shanghai 201203 (CN)
(72) Inventor: SHEN, Jingshan, Shanghai 201203 (CN); GONG, Xudong, Shanghai 201203 (CN); ZHU, Fuqiang, Shanghai 201200 (CN); JIANG, Xiangrui, Shanghai 201203 (CN); ZHANG, Yan, Shanghai 201203 (CN); SUN, Changliang, Shanghai 201203 (CN)
(74) Representative: Grünecker Patent- und Rechtsanwälte PartG mbB
(86) International application number: PCT/CN2020/090417
(87) International publication number: WO 2020/233502

(57) **Abstract**

Disclosed is a method for preparing cannabidiol and analogues thereof; the method is implemented by means of reacting a resorcinol derivative with menthyl-2,8-dien-1-ol or a derivative thereof. The method of the present invention has advantages of such as high chemical reaction selectivity and simple operation.

## Description

### Technical field

The present invention belongs to the field of chemical synthesis, and more specifically, relates to a method for chemically synthesizing cannabidiol or an analogue thereof.

### Background

In April 2005, Sativex^{®} (Cannabidiol/Dronabinol) developed by GW Pharmaceuticals was approved for marketing in Canada for the treatment of spasm caused by multiple sclerosis. In June 2018, cannabidiol was approved by the U.S. Food and Drug Administration (FDA) for the treatment of epilepsy caused by Lennox-Gastaut syndrome and Dravet syndrome, suitable for patients aged 2 years and above. In addition, the compound drug (Cannabidiol/Tetrahydrocannabivarin) comprising cannabidiol is currently in the phase II clinical trial and is expected to be used for the treatment of schizophrenia.

Methods for synthesizing Cannabidiol have been publicly reported. The first to appear was an alkylation reaction route in which 3,5-dihydroxypentbenzene was reacted with trans-menthyl-2,8-dien-1-ol under acid catalysis, but this route had poor selectivity, many impurities, difficulty in purification, low yield and other shortcomings.

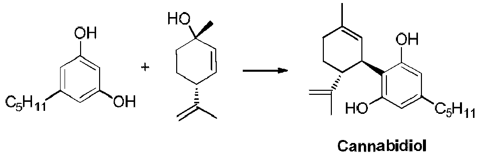

US2017008869 reported a reaction route in which 3,5-dihydroxypentylbenzene (Olivetol) was first halogenated, then reacted with trans-menthyl-2,8-dien-1-ol, and finally subjected to dehalogenation. However, the halogenation may generate isomers, and the final dehalogenation may lead to impurities.

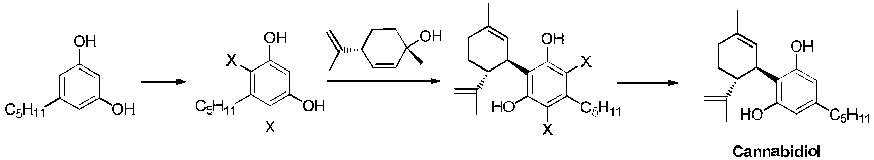

In addition, US5227537, EP2314580 etc. also publicly reported methods for synthesizing cannabidiol, in which purification methods such as rectification and column chromatography were used.

There are some shortcomings in the existing synthetic methods, such as poor selectivity of the reaction zone, cumbersome steps, and difficult separation and purification of the product. Therefore, it is necessary to develop a preparation method which has high reaction selectivity, simple operation and is suitable for industrial production.

### Summary of the invention

### Technical Problem

The technical problem to be solved by the present invention is to overcome the shortcomings of the prior art and provide a method for preparing cannabidiol or an analogue thereof that is mild in reaction conditions, simple in process, and suitable for industrialization.

Therefore, an object of the present invention is to provide a method for preparing cannabidiol or an analogue thereof, wherein the method is implemented by means of reacting a resorcinol or a derivative thereof with menthyl-2,8-dien-1-ol or a derivative thereof in the presence of a catalyst. The method has the advantages of high chemical reaction selectivity, simple operation, high product purity, etc., and is suitable for development as a large-scale production process.

### Technical Solution

According to one aspect, the present invention provides a method for preparing a compound of formula I, which is one of the following methods:

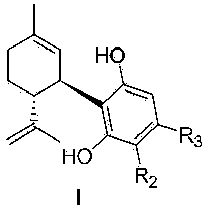

### Method One:

reacting a compound of formula II with a compound of formula III in the presence of a catalyst M to produce the compound of formula I,

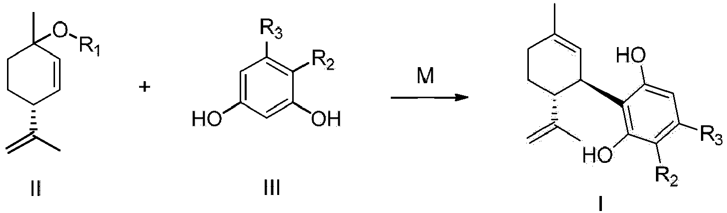

### Method Two:

reacting a compound of formula VI with a compound of formula III in the presence of a catalyst M to produce the compound of formula I, 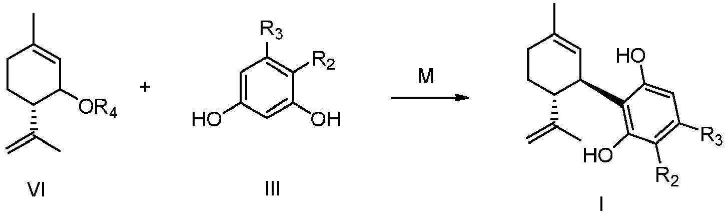 in formulas I, II, III and VI,
R₁ and R₄ are each independently hydrogen, carboxylic acyl (-(O=)CR₁ₐ), sulfonyl (-SO₂R_{1b}), substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; wherein R₁ₐ and R_{1b} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl, preferably, R₁ₐ and R_{1b} are each independently hydrogen, methyl, ethyl, propyl, phenyl, benzyl, phenethyl or phenylpropyl, wherein the substituent for the "substituted" is hydroxyl, amino, mercapto, an organophosphorus group, halogen, C₁-C₁₀ alkyl, C₃-C₁₀ cycloalkyl, or C₁-C₁₀ alkoxy,
further preferably, R₁ and R₄ are each independently hydrogen, methyl, ethyl, cyclopropylmethyl, methoxymethyl, 2-methoxyethyl, acetyl, propionyl, benzoyl, phenylacetyl, phenylpropionyl, methanesulfonyl, trifluoromethanesulfonyl or p-toluenesulfonyl,
R₂ is hydrogen, carboxyl, -COOR₅ or -CONR^{c}R^{d}; wherein R₅ is C₁-C₂₀ linear or branched alkyl, C₃-C₂₀ cycloalkyl, benzyl, or C₆-C₂₀ aryl; preferably, R₅ is methyl or ethyl; wherein R^{c} and R^{d} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; the substituent for the "substituted" is hydroxyl, amino, mercapto, an organophosphorus group, halogen, C₁-C₁₀ alkyl, C₃-C₁₀ cycloalkyl, or C₁-C₁₀ alkoxy; preferably, R^{c} and R^{d} are each independently hydrogen, methyl, ethyl or hydroxyethyl;
further preferably, R₂ is hydrogen, -COOH, -COOCH₃, -COOC₂H₅ or -(CH₂CH₂)₂NCH₃,
R₃ is substituted or unsubstituted C₁-C₂₀ linear or branched alkyl, substituted or unsubstituted C₃-C₂₀ cycloalkyl, substituted or unsubstituted C₃-C₂₀ alkenyl, substituted or unsubstituted C₃-C₂₀ alkynyl, substituted or unsubstituted C₃-C₂₀ acyl, substituted or unsubstituted C₆-C₂₀ aryl, or substituted or unsubstituted C₂-C₂₀ heteroaryl containing one or more atoms selected from the group consisting of oxygen, nitrogen, sulfur and phosphorus, wherein the substituent for the "substituted" is one or more selected from the group consisting of halogen; hydroxyl; C₁-C₂₀ alkyl; -O-C₁-C₂₀ alkyl; -NR^{a}R^{b}, wherein R^{a} and R^{b} are each independently selected from the group consisting of hydrogen and C₁-C₄ alkyl; -SO-C₁-C₂₀ alkyl; -SO₂-C₁-C₂₀ alkyl; C₃-C₂₀ alkenyl; C₃-C₂₀ alkynyl; C₁-C₂₀ acyl; C₆-C₂₀ aryl; C₂-C₂₀ heteroaryl containing one or more atoms selected from the group consisting of oxygen, nitrogen, sulfur and phosphorus; C₃-C₂₀ cycloalkyl; and C₂-C₂₀ heterocyclyl containing one or more atoms selected from the group consisting of oxygen, nitrogen, sulfur and phosphorus; preferably, R₃ is -C₅H₁₁.

In the method of the present invention, the compound of formula II or VI and the compound of formula III can be reacted in the presence of a catalyst M.

In one embodiment, R₁ and R₄ are each independently carboxylic acyl -(O=)CR₁ₐ, sulfonyl -SO₂R_{1b}, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl, the catalyst M may be one or more selected from the group consisting of Lewis acids, protic acids, acid anhydrides, silylesters, and silanes. Preferably, the Lewis acid may be selected from the group consisting of boron trihalides, more preferably, boron trichloride, boron trifluoride; aluminum trihalides, more preferably, aluminum trichloride, aluminum tribromide; transition metal salts, especially transition metal halides or trifluoromethanesulfonates, more preferably titanium tetrachloride, zinc chloride, zinc bromide, zinc trifluoromethanesulfonate; halides of the elements of the fourth, fifth, and sixth main groups in the periodic table of elements, more preferably, tin tetrachloride, phosphorus oxychloride, thionyl chloride, sulfone dichloride; the protic acid may be selected from the group consisting of perchloric acid, hydrogen halides, sulfuric acid, bisulfate, phosphoric acid, biphosphates, pyrophosphoric acid, R₆COOH and R₇SO₃H; wherein R₆ and R₇ may be each independently substituted or unsubstituted C₁-C₃₀ linear or branched alkyl, substituted or unsubstituted C₃-C₃₀ cycloalkyl, substituted or unsubstituted benzyl, or substituted or unsubstituted C₆-C₃₀ aryl, wherein the substituent for the "substituted" is one or more selected from the group consisting of carboxyl, phosphoric acid group, sulfonic acid group, phosphorous acid group and halogen; the acid anhydride may be one or more selected from the group consisting of trifluoromethanesulfonic anhydride, trifluoroacetic anhydride, methanesulfonic anhydride, ethanesulfonic anhydride, phenylmethanesulfonic anhydride, p-toluenesulfonic anhydride, trifluoroacetyl trifluoromethanesulfonate; the silylester may be one or two selected from the group consisting of trimethylsilyl trifluoromethanesulfonate and triethylsilyl trifluoromethanesulfonate; the silane may be one or more selected from the group consisting of trimethyliodosilane, triethyliodosilane, trimethylbromosilane, and trimethylchlorosilane.

In one embodiment, R₁ and R₄ are each independently hydrogen, and the catalyst M may be one or more selected from the group consisting of acid anhydrides, silylesters, and silanes. Preferably, the acid anhydride may be one or more selected from the group consisting of trifluoromethanesulfonic anhydride, trifluoroacetic anhydride, methanesulfonic anhydride, ethanesulfonic anhydride, phenylmethanesulfonic anhydride, and p-toluenesulfonic anhydride; the silylester may be one or two selected from the group consisting of trimethylsilyl trifluoromethanesulfonate and triethylsilyl trifluoromethanesulfonate; the silane may be one or more selected from the group consisting of trimethyliodosilane, triethyliodosilane, trimethylbromosilane and trimethylchlorosilane. The catalyst M may be one or more selected from the group consisting of methanesulfonic anhydride, trifluoromethanesulfonic anhydride, trimethylsilyl trifluoromethanesulfonate and trimethylsilyl iodide.

In the method of the present invention, preferably, the feeding molar ratio of the catalyst M to the compound of formula II or VI is 0.01:1 to 1:1, preferably 0.03:1 to 0.5:1, and more preferably 0.05 :1 to 0.5:1.

In one embodiment of the present invention, the compound of formula II or VI and the compound of formula III can be reacted in a solvent. The solvent may be one or a mixture of two or more selected from the group consisting of alkanes, aromatic hydrocarbons, halogenated hydrocarbons, esters, ethers and polar aprotic solvents.

Preferably, the alkane may be a C₅-C₂₀ linear or branched or cyclic alkane, including but not limited to n-pentane, n-hexane, n-heptane, cyclohexane, etc.

Preferably, the aromatic hydrocarbon may be a substituted benzene compound, including but not limited to toluene, xylene, chlorobenzene, etc.

Preferably, the halogenated hydrocarbon includes, but is not limited to, dichloromethane, 1,2-dichloroethane, chloroform, etc.

Preferably, the ester solvent includes, but is not limited to, methyl acetate, ethyl acetate, isopropyl acetate, butyl acetate, etc.

Preferably, the ether solvent includes, but is not limited to, tetrahydrofuran, dioxane, 2-methyltetrahydrofuran, diethyl ether, ethylene glycol dimethyl ether, cyclopentyl methyl ether, and methyl tert-butyl ether.

Preferably, the polar aprotic solvent includes, but is not limited to, acetonitrile, acetone, N,N-dimethylformamide, dimethylsulfoxide, N-methylpyrrolidone, and dimethylsulfoxide.

More preferably, the solvent is one or a mixture of two or more selected from n-heptane, n-hexane, toluene, xylene, chlorobenzene, dichloromethane, ethyl acetate, and tetrahydrofuran.

In the method of the present invention, preferably, the feeding molar ratio of the compound of formula II or VI to the compound of formula III is 1:5 to 5:1; more preferably, the molar ratio of the compound of formula II or VI to the compound of formula III is 0.5:1 to 2:1; further more preferably, the molar ratio of the compound of formula II or VI to the compound of formula III is 0.8:1 to 1.5:1.

In the method of the present invention, preferably, the feeding molar ratio of the catalyst to the compound of formula II or VI is 0.01:1 to 1:1; more preferably, the feeding molar ratio of the catalyst to the compound of formula II or VI is 0.03:1 to 0.5:1; further more preferably, the feeding molar ratio of the catalyst to the compound of formula II or VI is 0.05:1 to 0.5:1.

In the method of the present invention, preferably, the reaction is carried out at a temperature of -30°C to 50°C, more preferably, the reaction is carried out at a temperature of -20°C to 40°C.

In the method of the present invention, preferably, the reaction time of the reaction is usually 1h to 24h.

The method of the present invention may further comprise a step of purifying the compound of formula I. The step of purifying the compound of formula I can be carried out by layering.

The step of purifying the compound of formula I includes two routes:
Route (a):
   in the case that the reaction solvent is a polar aprotic solvent selected from the group consisting of acetonitrile, acetone, N,N-dimethylformamide, dimethylsulfoxide, N-methylpyrrolidone and dimethylsulfoxide, after the reaction between the compound of formula II or VI and the compound of formula III is completed, the reaction mixture is added with saturated sodium bicarbonate to quench the reaction, added with an aqueous inorganic alkali solution, and then with a solvent or a mixed solvent of two or more selected from the group consisting of hydrocarbon solvents and ether solvents for layering;
Route (b):
   in the case that the reaction solvent is one or a mixture of two or more selected from the group consisting of alkanes, aromatic hydrocarbons, halogenated hydrocarbons, esters and ethers other than the polar aprotic solvent, after the reaction between the compound of formula II or VI and the compound of formula III is completed, the reaction mixture is added with saturated sodium bicarbonate to quench the reaction, and then with water and layered, and the organic phase is concentrated to remove the solvent to obtain a crude product of formula I, which is added with an aqueous inorganic alkali solution, and then with a solvent or a mixed solvent of two or more selected from the group consisting of hydrocarbon solvents and ether solvents for layering.

In the step of layering and purification above, the solvent used for layering is a mixture of one or a mixed solvent of two or more selected from the group consisting of hydrocarbon solvents and ether solvents, and an aqueous inorganic alkali solution.

Preferably, the hydrocarbon solvent includes alkane solvents and unsaturated aromatic hydrocarbon solvents; the alkane solvent may be C₅-C₂₀ linear or branched or cyclic alkane; the unsaturated aromatic hydrocarbon solvent may be toluene, chlorobenzene, xylene, nitrobenzene.

Preferably, the ether solvent may be tetrahydrofuran, methyl tert-butyl ether, diethyl ether, cyclopentyl methyl ether, isopropyl ether, anisole, ethylene glycol dimethyl ether, tetrahydropyran, or dioxane.

Preferably, the inorganic alkali in the aqueous inorganic alkali solution may be one or a mixture of two or more selected from the group consisting of sodium hydroxide, potassium hydroxide, lithium hydroxide, sodium carbonate, potassium carbonate, and lithium carbonate; preferably, one of sodium hydroxide and potassium hydroxide or a mixture thereof; the mass fraction of the aqueous inorganic alkali solution is 1%-30%, preferably, 10%.

In the above step of purification, preferably, the step of purifying the compound of formula I further comprises: adjusting the pH of the aqueous phase to 6-7 with an acid after layering, adding an organic solvent to the aqueous phase for extraction, and concentrating the combined organic phase to dryness to obtain the compound of formula I.

Preferably, the acid used may be selected from the group consisting of organic acids and inorganic acids, wherein, the organic acid used is selected from the group consisting of formic acid, acetic acid, propionic acid, oxalic acid, maleic acid, citric acid, tartaric acid, methanesulfonic acid, benzenesulfonic acid, and wherein, the inorganic acid used is selected from the group consisting of hydrochloric acid, sulfuric acid, and phosphoric acid.

Preferably, the organic solvent is one or a mixture of two or more selected from the group consisting of 2-methyltetrahydrofuran, diethyl ether, ethylene glycol dimethyl ether, a C₅-C₂₀ linear or branched or cyclic alkane, benzene, toluene, xylene, dichloromethane, 1,2-dichloroethane, chloroform, cyclopentyl methyl ether, and methyl tert-butyl ether, preferably one or a mixture of two or more selected from the group consisting of dichloromethane, tetrahydrofuran, toluene, xylene, chlorobenzene, n-heptane, and n-hexane.

The present invention also relates to a method for preparing a compound of formula V, comprising:
using the method for preparing the compound of formula I of the present invention to obtain the compound of formula I,
removing R₂ from the compound of formula I to produce a compound of formula V, 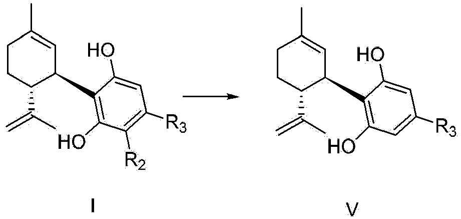 wherein, R₂ is carboxyl, alkoxycarbonyl (-COOR₅) or aminocarbonyl (-CONR^{c}R^{d});
wherein R₅ is C₁-C₂₀ linear or branched alkyl, C₃-C₂₀ cycloalkyl, benzyl, or C₆-C₂₀ aryl; preferably, R₅ is methyl or ethyl; and
wherein R^{c} and R^{d} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; the substituent for the "substituted" is hydroxyl, amino, mercapto, an organophosphorus group, halogen, C₁-C₁₀ alkyl, C₃-C₁₀ cycloalkyl, or C₁-C₁₀ alkoxy; preferably, R^{c} and R^{d} are each independently hydrogen, methyl, ethyl or hydroxyethyl;
preferably, R₂ is hydrogen, -COOH, -COOCH₃, -COOC₂H₅ or -(CH₂CH₂)₂NCH₃,
R₃ is substituted or unsubstituted C₁-C₂₀ linear or branched alkyl, substituted or unsubstituted C₃-C₂₀ cycloalkyl, substituted or unsubstituted C₃-C₂₀ alkenyl, substituted or unsubstituted C₃-C₂₀ alkynyl, substituted or unsubstituted C₃-C₂₀ acyl, substituted or unsubstituted C₆-C₂₀ aryl, or substituted or unsubstituted C₂-C₂₀ heteroaryl containing one or more atoms selected from the group consisting of oxygen, nitrogen, sulfur and phosphorus, wherein the substituent for the "substituted" is one or more selected from the group consisting of halogen; hydroxyl; C₁-C₂₀ alkyl; -O-C₁-C₂₀ alkyl; -NR^{a}R^{b} (wherein R^{a} and R^{b} are each independently selected from the group consisting of hydrogen and C₁-C₄ alkyl); -SO-C₁-C₂₀ alkyl; -SO₂-C₁-C₂₀ alkyl; C₃-C₂₀ alkenyl; C₃-C₂₀ alkynyl; C₁-C₂₀ acyl; C₆-C₂₀ aryl; C₂-C₂₀ heteroaryl containing one or more atoms selected from the group consisting of oxygen, nitrogen, sulfur and phosphorus; C₃-C₂₀ cycloalkyl; and C₂-C₂₀ heterocyclyl containing one or more atoms selected from the group consisting of oxygen, nitrogen, sulfur and phosphorus; preferably, R₃ is -C₅H₁₁.

The reaction of removing R₂ from the compound of formula I to produce the compound of formula V is carried out in the presence of one or a mixture of two or more selected from the group consisting of alkalis, and alkali metal or alkaline earth metal salts;
wherein preferably, the alkali is one or a mixture of two or more selected from the group consisting of alkali metal or alkaline earth metal hydroxides, alkali metal or alkaline earth metal carbonates, alkali metal or alkaline earth metal alkoxides, and nitrogen-containing organic bases, more preferably one or a mixture of two or more selected from the group consisting of sodium hydroxide, potassium hydroxide, lithium hydroxide, sodium carbonate, potassium carbonate, lithium carbonate, sodium methoxide, potassium methoxide, lithium ethoxide, sodium ethoxide, magnesium methoxide, magnesium ethoxide, magnesium n-propoxide, calcium methoxide, calcium ethoxide, sodium tert-butoxide, lithium tert-butoxide, magnesium tert-butoxide, magnesium isobutoxide, magnesium tert-amyloxide, N-methylmorpholine, N,N-diisopropylethylamine, triethylamine, tripropylamine, tri-n-propylamine, triisopropylamine, tributylamine, pyridine, pyrimidine, quinoline, N-methylpiperidine, N-methylpiperazine, imidazole, dimethylaminopyridine, N-methylmorpholine, dimethylaniline, 1,8-diazabicyclo[5.4.0]undec-7-ene (DBU), 1,5-diazabicyclo[4.3.0]non-5-ene (DBN), and 1,4-diazabicyclo[2.2.2]octane (DABCO); preferably, the alkali is selected from the group consisting of sodium hydroxide, potassium hydroxide, lithium hydroxide, sodium carbonate, potassium carbonate, lithium carbonate, magnesium ethoxide, magnesium n-propoxide, magnesium tert-butoxide and magnesium isobutoxide; more preferably, the alkali is sodium hydroxide, potassium hydroxide, lithium hydroxide, sodium carbonate, potassium carbonate, lithium carbonate, magnesium ethoxide, or magnesium tert-butoxide;
wherein, preferably, the alkali metal or alkaline earth metal salt is one or a mixture of two or more selected from the group consisting of alkali metal or alkaline earth metal halides, alkali metal or alkaline earth metal carboxylates, and alkali metal or alkaline earth metal sulfonates; more preferably the alkali metal or alkaline earth metal salt is selected from the group consisting of lithium chloride, lithium bromide, lithium iodide, lithium acetate, lithium trifluoroacetate, lithium benzoate, lithium trifluoromethanesulfonate, lithium methanesulfonate, lithium phenylmethanesulfonate; sodium chloride, sodium bromide, sodium iodide, sodium acetate, sodium trifluoroacetate, sodium benzoate, sodium trifluoromethanesulfonate, sodium methanesulfonate, sodium phenylmethanesulfonate; potassium chloride, potassium bromide, potassium iodide, potassium acetate, potassium trifluoroacetate, potassium benzoate, potassium trifluoromethanesulfonate, potassium methanesulfonate, potassium phenylmethanesulfonate; magnesium chloride, magnesium bromide, magnesium iodide, magnesium acetate, magnesium trifluoroacetate, benzoic acid magnesium, magnesium trifluoromethanesulfonate, magnesium methanesulfonate, magnesium phenylmethanesulfonate, calcium chloride, calcium bromide, calcium acetate, calcium trifluoroacetate, calcium benzoate; preferably, the alkali metal or alkaline earth metal salt is lithium bromide, lithium chloride, sodium chloride, potassium chloride, magnesium bromide, or magnesium chloride; more preferably, the alkali metal or alkaline earth metal salt is magnesium chloride or lithium chloride.

A solvent is used to carry out the reaction, preferably, the solvent is one or a mixture of two or more selected from the group consisting of methanol, ethanol, isopropanol, ethylene glycol, tetrahydrofuran, dioxane, 2-methyltetrahydrofuran, diethyl ether, ethylene glycol dimethyl ether, cyclopentyl methyl ether, methyl tert-butyl ether, dimethyl sulfoxide, N-methylpyrrolidone, N,N-dimethylformamide, N,N-dimethylacetamide, N,N-diethylformamide, N,N-diethylacetamide and water.

In the method of the present invention, preferably, the feeding molar ratio of the alkali metal or alkaline earth metal salt to the compound of formula I is 20:1 to 1:4, preferably 15:1 to 1:2, more preferably 10:1 to 1:1; and the feeding molar ratio of the alkali to the compound of formula I is 10:1 to 1:4; preferably 8:1 to 1:3, more preferably 5: 1 to 1:1.

Preferably, the method of preparing the compound of formula V may further include a step of purifying the compound of formula V, wherein the step of purifying the compound of formula V comprises adding a solvent to the crude product of the compound of formula V for layering, preferably the solvent used for layering is a mixture of one or a mixed solvent of two or more selected from the group consisting of alkane solvents, ether solvents and unsaturated aromatic hydrocarbon solvents, and an aqueous inorganic alkali solution. Preferably, the alkane solvent may be a C₅-C₂₀ linear or branched or cyclic alkane. The unsaturated aromatic hydrocarbon solvent may be selected from the group consisting of toluene, chlorobenzene, and xylene; the ether solvent is one or a mixture of two or more selected from the group consisting of tetrahydrofuran, methyl tert-butyl ether, diethyl ether, cyclopentyl methyl ether, isopropyl ether, anisole, ethylene glycol dimethyl ether, tetrahydropyran, and dioxane.

Preferably, the inorganic alkali in the aqueous inorganic alkali solution is one or a mixture of two or more selected from sodium hydroxide, potassium hydroxide, lithium hydroxide, sodium carbonate, potassium carbonate, and lithium carbonate; preferably, one or two selected from the group consisting of sodium hydroxide and potassium hydroxide; the mass fraction of the aqueous inorganic alkali solution is 1%-30%, preferably, 10%.

Preferably, after the completion of layering, the pH of the organic phase is adjusted to 6-7 with an acid, and the organic phase is concentrated to dryness to obtain an oily compound of formula V.

Preferably, the step of purifying the compound of formula V further comprises: adjusting the pH of the organic phase to 6-7 with an acid after completion of layering, and after layering, concentrating the organic phase to dryness to obtain an oily compound of formula V;
wherein, preferably, the acid used is one of organic acids and inorganic acids, wherein, more preferably, the organic acid used is selected from the group consisting of formic acid, acetic acid, propionic acid, oxalic acid, maleic acid, citric acid, tartaric acid, methanesulfonic acid, and benzenesulfonic acid, and wherein, further more preferably, the inorganic acid used is selected from the group consisting of hydrochloric acid, sulfuric acid, and phosphoric acid.

Preferably, the organic solvent is one or a mixture of two or more selected from the group consisting of 2-methyltetrahydrofuran, diethyl ether, ethylene glycol dimethyl ether, C₅-C₂₀ linear or branched or cyclic alkane, benzene, toluene, xylene, dichloromethane, 1,2-dichloroethane, chloroform, cyclopentyl methyl ether, and methyl tert-butyl ether, preferably selected from the group consisting of dichloromethane, tetrahydrofuran, toluene, xylene, chlorobenzene, n-heptane, and n-hexane.

According to another aspect, the present invention provides a crystal form A of cannabidiol, and the DSC spectrum data of the crystal form A are as follows:
Onset = 65.46±1°C, peak = 68.66±1°C.

The X-ray powder diffraction data of the crystal form A are as follows: there are X-ray diffraction peaks at 2θ of 5.097°±0.2°, 9.40°±0.2°, 9.71°±0.2°, 1022°±0.2°, 11.79°±0.2°, 12.503°±0.2°, 13.147°±0.2°, 13.787°±0.2°, 15.086°±0.2°, 17.05°±0.2°, 17.40°±0.2°, 17.98°±0.2°, 19.00°±0.2°, 19.83°±0.2°, 20.891°±0.2°, 21.685°±0.2°, 22.17°±0.2°, 22.60°±0.2°, 24.416°±0.2°, 29.091°±0.2°, and 31.133°±0.2°.

According to another aspect, the present invention provides a method for preparing the crystal form A of cannabidiol, comprising: dissolving cannabidiol in an alkane solvent in amount of 0.2-10 times of the weight of the cannabidiol, cooling to a temperature of -50°C to 10°C, stirring or standing at the temperature, and then filtering or centrifuging the suspension to separate and obtain the crystal form A of cannabidiol. Preferably, the preparation method is carried out in the presence of the seed crystal of the crystal form A.

The alkane is one or a mixture of two or more selected from the group consisting of C₄-C₂₀ linear or branched or cyclic alkanes, preferably, one or a mixture of two or more selected from the group consisting of n-heptane, petroleum ether, cyclopentane, and cycloheptane.

In the method for preparing a crystal form A of cannabidiol above, the cannabidiol is prepared by the aforementioned method of the present invention.

According to still another aspect, the present invention provides a method for preparing a single crystal of the crystal form A of cannabidiol, comprising: dissolving cannabidiol in an alkane solvent in amount of 0.2-10 times of the weight of the cannabidiol, cooling to a temperature of -30°C to -10°C, standing at the temperature, and then filtering or centrifuging the suspension to separate and obtain the single crystal of the crystal form A of cannabidiol. Preferably, the preparation method is carried out in the presence of the seed crystal of the crystal form A.

The alkane is one or a mixture of two or more selected from the group consisting of C₄-C₂₀ linear or branched or cyclic alkanes; preferably one or a mixture of two or more selected from the group consisting of n-heptane, petroleum ether and n-octane. The space group of the single crystal of the crystal form A of cannabidiol is a monoclinic crystal system, the axial length a=10.403 (Å), b=10.894 (Å), c=16.692 (Å), and the angle between the crystal planes α=90°, β=95.49°, γ=90°, showing the same chemical structure as that of natural sources (Acta Cryst. (1977). B33, 3211-3214).

In the method for preparing the single crystal of the cannabidiol crystal form A, preferably, the cannabidiol is prepared by the aforementioned method of the present invention.

### Beneficial Effect

The method disclosed in the present invention is superior to the existing methods. The method disclosed by the invention has the advantages of high chemical reaction selectivity, simple operation, high product purity and the like.

### Description of the drawings

Figure 1 is an X-ray powder diffraction pattern of cannabidiol crystal form A;
Figure 2 is a single crystal diagram of cannabidiol crystal form A;
Figure 3 is a liquid chromatogram of cannabidiol; and
Figure 4 is a differential scanning calorimetry analysis diagram of cannabidiol crystal form A.

### Mode of the Invention

The invention discloses preparation and purification methods of cannabidiol or an analogue thereof. For example, the present invention discloses a method for preparing cannabidiol or an analogue thereof. The following examples are only used to illustrate the embodiments of the present invention. It should be understood that the embodiments of the present invention are not limited to the specific details in the following examples, because in view of the disclosure of the present invention, other variations are known and obvious to an ordinary skilled in the art.

The measurement conditions of X-ray powder diffraction pattern are as follows:
Instrument model: Bruker D8 advance, target: Cu Kα (40kV, 40mA), distance from sample to detector: 30 cm, scan type: locked coupled, step width: 0.02°, scan range: 3°-40° (2θ value), scan step time: 0.1 s.

### Example 1

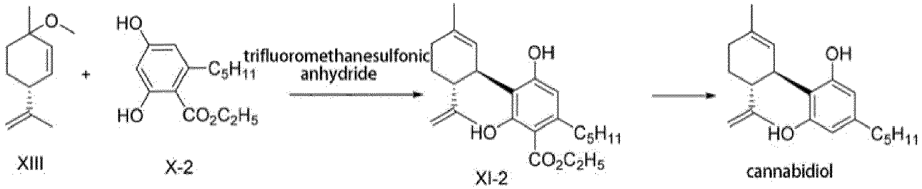

X-2 (1.0 g, 3.76 mmol) was dissolved in chlorobenzene, dropped slowly with trifluoromethanesulfonic anhydride (1.0 g, 0.08 eq) at -10°C to 0°C, followed by dropwise addition of XIII (681 mg, 1.1 eq) at -10°C to 0°C. TLC showed that the reaction was almost complete. The reaction mixture was added with saturated sodium bicarbonate to quench the reaction, and then added with water and layered. The organic phase was concentrated to a small volume and added with cyclohexane, chlorobenzene, methanol and a 10% LiOH aqueous solution and layered. The lower layer was adjusted with maleic acid to pH=6-7, filtered and layered with cyclohexane, and the organic phase was concentrated to obtain 1.36g of compound XI-2.

¹H NMR (DMSO, 400 MHz): 0.87 (t, 3H), 1.24-1.35 (m, 7H), 1.43-1.48 (m, 2H), 1.59 (s, 3H), 1.61 (s, 3H), 1.64-1.72 (m, 2H), 1.92-1.96 (m, 1H), 2.09-2.16 (m, 1H), 2.70 (t, 2H), 3.00-3.06(m, 1H), 3.89-3.92(m, 1H), 4.32 (q, 2H), 4.42(d, 1H), 4.60(d, 1H), 5.09 (s, 1H), 6.20 (s, 1H), 9.92 (s, 1H), 11.80 (s, 1H). LR-MS (ESI) *m*/*z:* 385 (M-H)⁻.

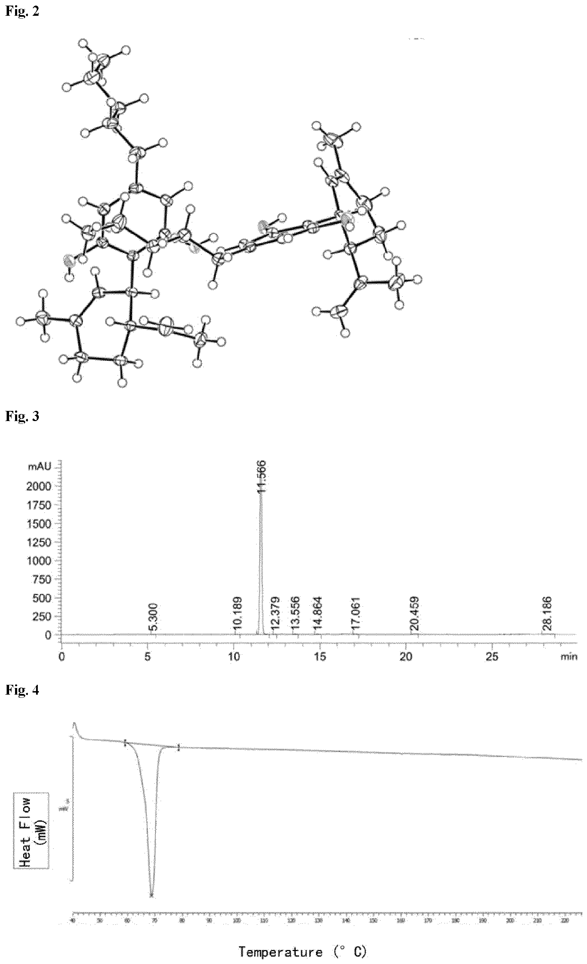

The above XI-2 was dissolved in methanol, added with a 10% LiOH aqueous solution and heated to reflux. After TLC showed that XI-2 was completely reacted, the reaction mixture was added with petroleum ether and layered. The petroleum ether phase was adjusted to pH 6-7 with saturated tartaric acid aqueous solution, and concentrated to dryness to remove petroleum ether to obtain 0.9 g of a light brown oil. The light brown oil was dissolved in n-heptane, and stirred at -10°C to 0°C for 4h. A large amount of solids were precipitated out, and filtered to obtain 700 mg of cannabidiol in crystal form A as a white solid with a yield in two steps of 54.2% and a HPLC purity of 99.5%. Figure 3 shows a liquid chromatogram of the cannabidiol prepared by this method.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H),4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

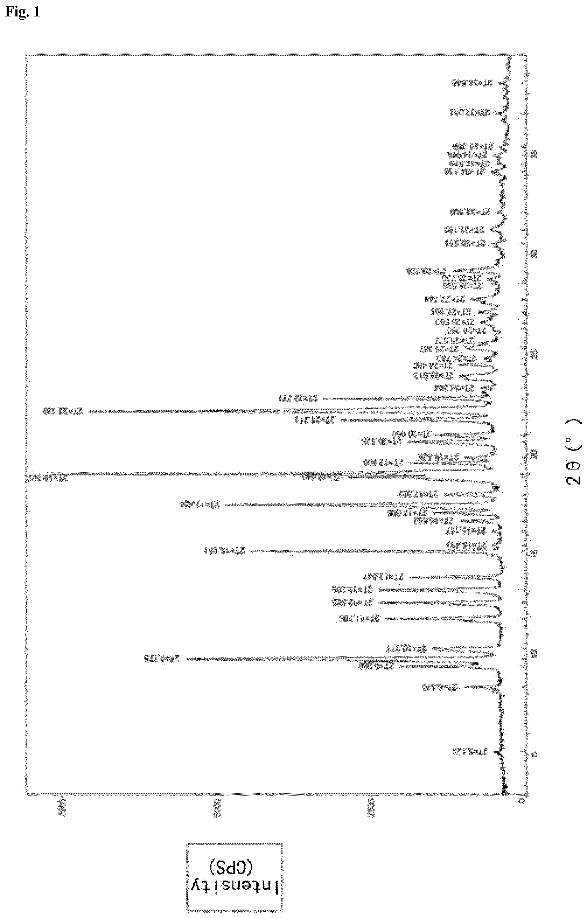

The X-ray powder diffraction pattern of the cannabidiol crystal form A was determined, and the result was shown in Figure 1.

According to Figure 1, the crystal form A has X-ray diffraction peaks at the diffraction angle 2θ of 5.097°±0.2°, 9.40°±0.2°, 9.71°±0.2°, 10.22°±0.2°, 11.79°±0.2°, 12.503°±0.2°, 13.147° ±0.2°, 13.787°±0.2°, 15.086°±0.2°, 17.05°±0.2°, 17.40°±0.2°, 17.98°±02°, 19.00°±0.2°, 19.83°±0.2°, 20.891°±0.2°, 21.685°±0.2°, 22.17°±0.2°, 22.60°±0.2°, 24.416°±0.2°, 29.091°±0.2°, 31.133°±0.2°.

1 g of the white solid was dissolved in n-heptane under heating, and kept at -30°C to -20°C for 36h. A large amount of crystals were formed, and 500 mg of a single crystal of crystal form A was obtained by filtration. Figure 2 showed an image of the single crystal of the cannabidiol crystal form A.

The space group of the single crystal of the crystal form A is a monoclinic crystal system with an axial lengths a=10.403 (Å), b=10.894 (Å), c=16.692 (Å), and an angles between the crystal planes α=90°, β=95.49°, γ=90°.

Figure 4 showed a graph of differential scanning calorimetry analysis of the cannabidiol crystal form A.

### Example 2

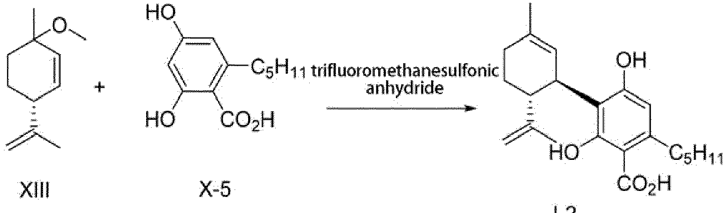

X-5 (1.0 g, 5.6 mmol) was dissolved in dichloromethane/toluene, added with anhydrous magnesium sulfate, and dropped slowly with a solution of trifluoromethanesulfonic anhydride (0.15 g, 0.1 eq) in toluene at -10°C to 0°C, followed by dropwise addition of XIII (938 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, methanol and a 10% KOH aqueous solution and layered. The lower layer was adjusted with a saturated tartaric acid aqueous solution to pH=5-6, and layered with dichloromethane. The organic phase was concentrated to dryness to obtain 700 mg of the product with a total yield of 43.7%.

¹H NMR (CDCl₃, 400 MHz) :0.90 (3H, m), 1.20- 1.33 (4H, m), 1.55 (2H, m), 1.72 (3H s), 1.80 (3H, s), 2.01- 2.39(5H, m), 2.92 (2H, t), 4.10 (1H, m), 4.40 (1H, s), 4.55 (1H, s), 5.57(1H, s), 6.26(1H, s), 6.66(2H, s), 11.85 (1H, s). LR-MS (ESI) *m*/*z:* 357 (M-H)⁻.

### Example 3

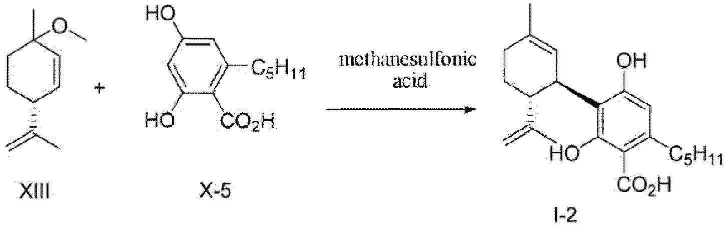

X-5 (1.0 g, 5.6 mmol) was dissolved in chloroform, added with anhydrous magnesium sulfate, and dropped slowly with a solution of methanesulfonic acid (85 mg, 0.2 eq) in toluene at -10°C to 0°C, followed by dropwise addition of XIII (938 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with petroleum ether, methanol, and a 10% KOH aqueous solution and layered. The lower layer was adjusted with a saturated tartaric acid aqueous solution to pH=5-6, and layered with n-heptane. The organic phase was concentrated to dryness to obtain 600 mg of the product with a total yield of 37.4%.

The NMR spectrum of this compound was measured, and it was the same as the product of Example 2.

### Example 4

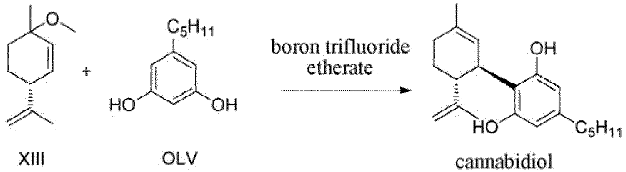

OLV (1.0 g, 5.6 mmol) was dissolved in dichloromethane/toluene, added with anhydrous magnesium sulfate, and dropped slowly with a solution of boron trifluoride etherate (0.15 g, 0.1 eq) in toluene at -10°C to 0°C, followed by dropwise addition of XIII (1.02 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, methanol, and a 10% KOH aqueous solution and layered. The organic phase was adjusted with a saturated tartaric acid aqueous solution to pH=6-7, concentrated to dryness to obtain 800 mg of a light brown oil. The above oil was added with petroleum ether, and stirred for 12 h at -20°C. A large amount of solids were precipitated out, filtered and dried to obtain 500 mg of the product with a total yield of 28.4%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 5

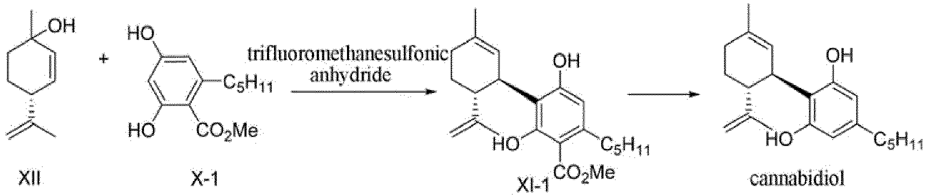

X-1 (1 g, 4.2 mmol) was dissolved in dichloromethane, added with trifluoromethanesulfonic anhydride (0.1 g, 0.08 eq) in dichloromethane at -10°C to 0°C, followed by addition of XII (702 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile, and a 10% KOH aqueous solution, stirred and layered. The aqueous phase was added with maleic acid to adjust pH=6-7 and layered with dichloromethane, and the organic phase was concentrated to obtain 1.3 g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in methanol, added with a 10% KOH aqueous solution, and heated to reflux. TLC showed that XI-1 was completely reacted. The reaction mixture was added with methyl tert-butyl ether and layered. The methyl tert-butyl ether phase was adjusted to pH 6-7 with a saturated tartaric acid aqueous solution, and concentrated to remove methyl tert-butyl ether to obtain 0.9 g of a light brown oil. The light brown oil was dissolved in cycloheptane, and stirred at -20°C for 12h. A large amount of solids were precipitated out, filtered and dried to obtain 680 mg of cannabidiol in crystal form A as a white solid with a yield in two steps of 53.4% and a HPLC purity of 99.5%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

The X-ray powder diffraction pattern of the cannabidiol crystal form A was measured, and the result was the same as that in Example 1.

1 g of the white solid was dissolved in n-heptane under heating, and kept at -30°C to -40°C for 48h. A large amount of crystals were formed, and 500 mg of single crystal of the crystal form A was obtained by filtration. The analysis result of the single crystal of the crystal form A was the same as that in Example 1.

### Example 6

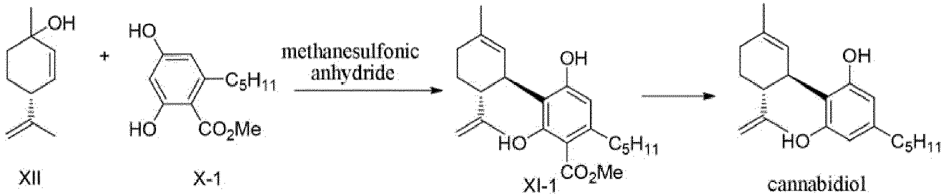

X-1 (1.0 g, 4.2 mmol) was dissolved in dichloromethane, added with anhydrous magnesium sulfate, and dropped slowly with a solution of methanesulfonic anhydride (0.21 g, 0.3 eq) in dichloromethane at -10°C to 0°C, followed by dropwise addition of XII (0.72 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, methyl tert-butyl ether, N,N-dimethylformamide and a 10% LiOH aqueous solution and layered. The lower layer was adjusted to pH=6-7 with fumaric acid and layered with ethyl acetate, and the organic phase was concentrated to obtain 1.12 g of XI-1.

The above XI-1 was dissolved in N,N-dimethylacetamide, added with anhydrous magnesium chloride (800 mg, 2 eq) and N,N-diisopropylethylamine (1.08 g, 2 eq), and heated to 128°C to react overnight. The reaction mixture was cooled down to room temperature, added with diethyl ether and layered. The diethyl ether phase was adjusted to pH=6-7 with a saturated tartaric acid aqueous solution, concentrated to dryness to remove diethyl ether to obtain 0.85 g of a light brown oil. The above light brown oil was added with cyclohexane and stirred at -20°C for 12h. A large amount of solids were precipitated out, filtered and dried to obtain 580 mg of cannabidiol in crystal form A with a yield in two steps of 45.2%.

The NMR spectrum of this compound was measured, and it was the same as the product of Example 5.

### Example 7

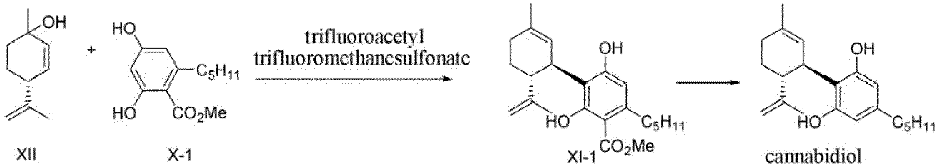

X-1 (1.0 g, 4.2 mmol) was dissolved in tetrahydrofuran, dropped slowly with a solution of trifluoroacetyl trifluoromethanesulfonate (0.11 g, 0.1 eq) in tetrahydrofuran at -10°C to 0°C, followed by dropwise addition of XII (0.702 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-hexane, cyclopentyl methyl ether, methanol and a 10% KOH aqueous solution and layered. The lower layer was adjusted with tartaric acid to pH=6-7 and layered with isopropyl acetate, and the organic phase was concentrated to obtain 1.38 g of XI-1.

The above XI-1 was dissolved in tetrahydrofuran, added with a 10% NaOH aqueous solution, and heated to reflux. TLC showed that XI-1 was reacted completely. The reaction mixture was added with toluene and layered. The toluene phase was adjusted to pH 6-7 with saturated tartaric acid aqueous solution, and concentrated to dryness to remove toluene to obtain 0.81 g of a light brown oil. The above light brown oil was added with n-hexane and stirred at -20°C for 12h. A large amount of solids were precipitated out, filtered and dried to obtain 550 mg of cannabidiol in crystal form A with a yield in two steps of 42.6%.

The NMR spectrum of this compound was measured, and it was the same as the product of Example 5.

### Example 8

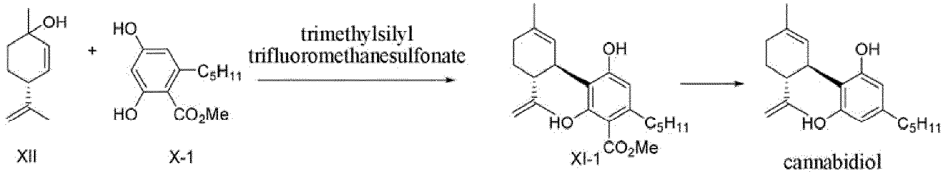

X-1 (1.0 g, 4.2 mmol) was dissolved in n-heptane, dropped slowly with a solution of trimethylsilyl trifluoromethanesulfonate (0.1 g, 0.1 eq) in toluene at -10°C to 0°C, followed by dropwise addition of XII (702 mg, 1.leq) at -10°C to 0°C. TLC showed that the reaction was almost complete. The reaction mixture was added with saturated sodium bicarbonate to quench the reaction, added with water and layered. The organic phase was concentrated to a small volume, added with xylene, n-heptane, dimethyl sulfoxide and a 10% LiOH aqueous solution and layered. The aqueous phase was adjusted to pH=6-7 with citric acid and layered with chloroform, and the organic phase was concentrated to obtain 1.44g of XI-1.

The above XI-1 was dissolved in ethylene glycol, added with lithium chloride (356 mg, 2 eq) and N,N-diisopropylethylamine (1.08 g, 2 eq), and heated to 128°C to react overnight. The reaction mixture was cooled down to room temperature, added with isopropyl ether and layered. The isopropyl ether phase was adjusted to pH=6-7 with saturated tartaric acid aqueous solution, concentrated to dryness to remove isopropyl ether to obtain 0.9 g of a light brown oil. The above light brown oil was added with cyclohexane and stirred at -20°C for 12h. A large amount of solids were precipitated out, filtered and dried to obtain 700 mg of cannabidiol in crystal form A with a yield in two steps of 54.2%.

The NMR spectrum of this compound was measured, and it was the same as the product of Example 5.

### Example 9

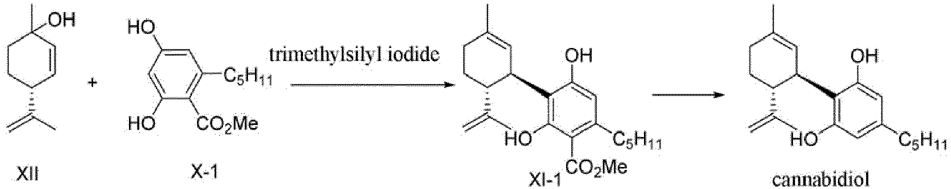

X-1 (1.0 g, 4.2 mmol) was dissolved in methyl tert-butyl ether, added with sodium sulfate, dropped slowly with a solution of trimethylsilyl iodide (0.84 g, 0.1 eq) in methyl tert-butyl ether at -10°C to 0°C, followed by dropwise addition of XII (702 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, N,N-methylacetamide and a 10% NaOH aqueous solution and layered. The lower layer was adjusted to pH=6-7 with maleic acid and layered with cyclohexane, and the organic phase was concentrated to obtain 1.29 g of XI-1.

The above XI-1 was dissolved in acetonitrile, added with a 10% LiOH aqueous solution, and heated to reflux. TLC showed that XI-1 was reacted completely. The reaction mixture was added with n-hexane and layered. The n-hexane phase was adjusted to pH 6-7 with saturated tartaric acid aqueous solution, concentrated to dryness to remove n-hexane to obtain 0.9 g of a light brown oil. The above light brown oil was added with n-hexane and stirred at -20°C for 12h. A large amount of solids were precipitated out, filtered and dried to obtain 600 mg of cannabidiol in crystal form A with a yield in two steps of 46.4%.

The NMR spectrum of this compound was measured, and it was the same as the product of Example 5.

### Example 10

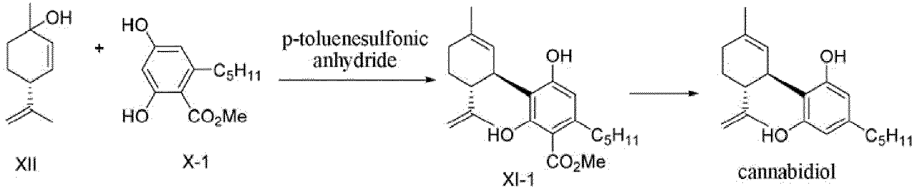

X-1 (1.0g, 4.2mmol) was dissolved in 1,2-dichloroethane, dropped slowly with a solution of p-toluenesulfonic anhydride (1.36g, 0.1eq) in 1,2-dichloroethane at -10°C to 0°C, followed by dropwise addition of XII (702mg, 1.leq) at -10°C to 0°C. TLC showed that the reaction was almost complete. The reaction mixture was added with saturated sodium bicarbonate to quench the reaction, added with water and layered. The organic phase was concentrated to a small volume, added with petroleum ether, methanol and a 10% KOH aqueous solution and layered. The lower layer was adjusted to pH=6-7 with maleic acid, filtered and layered with n-octane, and the organic phase was concentrated to obtain 1.36 g of XI-1 as an oil.

The above XI-1 was dissolved in toluene, added with a 10% LiOH aqueous solution, and heated to reflux. TLC showed that XI-1 was reacted completely. The reaction mixture was added with n-octane and layered. The n-octane phase was adjusted to pH 6-7 with saturated tartaric acid aqueous solution, concentrated to dryness to remove n-octane to obtain 0.9 g of a light brown oil. The above light brown oil was added with n-octane and stirred at -20°C for 12h. A large amount of solids were precipitated out, filtered and dried to obtain 610 mg of cannabidiol in crystal form A with a yield in two steps of 47.1%.

The NMR spectrum of this compound was measured, and it was the same as the product of Example 5.

### Example 11

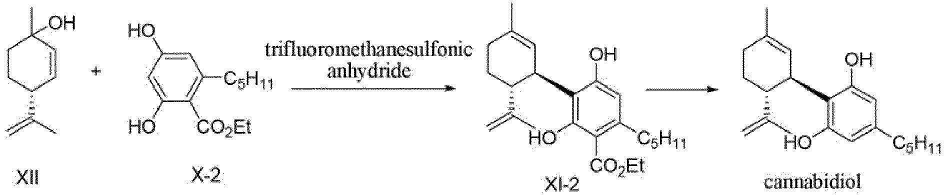

X-2 (1.0 g, 3.76 mmol) was dissolved in chlorobenzene, dropped slowly with a solution of trifluoromethanesulfonic anhydride (1.0 g, 0.08 eq) in chlorobenzene at -10°C to 0°C, followed by dropwise addition of XII (0.629 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, xylene, methanol and a 10% LiOH aqueous solution and layered. The lower layer was adjusted to pH=6-7 with maleic acid, filtered and layered with n-heptane, and the organic phase was concentrated to obtain 1.36 g of XI-2.

The above XI-2 was dissolved in dimethyl sulfoxide, added with magnesium tert-butoxide (1.28 g, 2 eq), and heated to reflux. TLC showed that XI-1 was reacted completely. The reaction mixture was added with petroleum ether and layered. The petroleum ether phase was adjusted to pH 6-7 with saturated tartaric acid aqueous solution, concentrated to dryness to remove petroleum ether to obtain 0.9 g of a light brown oil. The above light brown oil was added with n-heptane and stirred at -10°C to 0°C for 4h. A large amount of solids were precipitated out, and filtered to obtain 850 mg of white solids. The white solids above were added with cyclopentane and stirred at -20°C for 12h. A large amount of solids were precipitated out, filtered and dried to obtain 700mg of cannabidiol in crystal form A with a yield in two steps of 54.2%.

The NMR spectrum of this compound was measured, and it was the same as the product of Example 1.

### Example 12

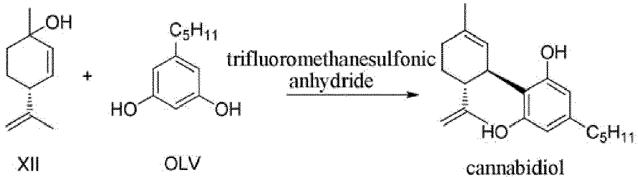

OLV (1.0 g, 5.6 mmol) was dissolved in dichloromethane/toluene, added with anhydrous magnesium sulfate, and dropped slowly with a solution of trifluoromethanesulfonic anhydride (0.15 g, 0.1 eq) in toluene at -10°C to 0°C, followed by dropwise addition of XII (938 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, methanol, and a 10% KOH aqueous solution and layered. The organic phase was adjusted to pH=6-7 with saturated tartaric acid aqueous solution, concentrated to dryness to obtain 1.36 g of a light brown oil. The above light brown oil was added with n-heptane and stirred at -20°C for 12h. A large amount of solids were precipitated out, filtered and dried to obtain 900 mg of a light white solid with a purity of 90%. The above solid was dissolved in n-heptane and stirred at -20°C for 12h. A large amount of solids were precipitated out, filtered and dried to obtain 700 mg of the product with a total yield of 39.7%.

The NMR spectrum of this compound was measured, and it was the same as the product of Example 4.

### Example 13

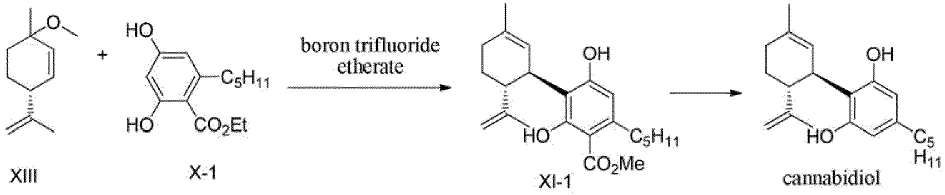

X-1 (1 g, 4.2 mmol) was dissolved in dichloromethane/toluene, dropped slowly with boron trifluoride etherate (60mg, 0.1eq) at -10°C to 0°C, followed by dropwise addition of XIII (769 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, xylene, acetonitrile and a 10% KOH aqueous solution and layered. The lower layer was adjusted to pH=6-7 with maleic acid and layered with n-heptane, and the organic phase was concentrated to obtain 1.5 g of XI-1.

The above XI-1 was dissolved in methanol, added with a 10% KOH aqueous solution, and heated to reflux. TLC showed that XI-1 was reacted completely. The reaction mixture was cooled down to room temperature, added with cyclopentyl methyl ether and layered. The cyclopentyl methyl ether phase was adjusted to pH 6-7 with saturated tartaric acid aqueous solution, concentrated to dryness to remove cyclopentyl methyl ether to obtain 1.1 g of a light brown oil. The above light brown oil was dissolved in n-hexane and stirred at -10°C to 0°C for 4h. A large amount of solids were precipitated out, filtered and dried to obtain 850 mg of cannabidiol in crystal form A with a yield in two steps of 66.5%.

The NMR spectrum of this compound was measured, and it was the same as the product of Example 5.

### Example 14

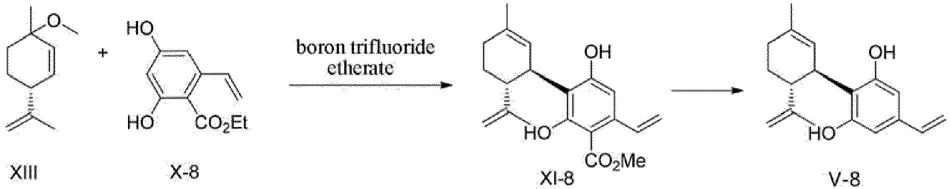

X-8 (1 g, 4.8 mmol) was dissolved in dichloromethane/toluene, dropped slowly with boron trifluoride etherate (65 mg, 0.1 eq) at -10°C to 0°C, followed by dropwise addition of XIII (820 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, xylene, acetonitrile and a 10% KOH aqueous solution and layered. The lower layer was adjusted to pH=6-7 with maleic acid and layered with n-heptane, and the organic phase was concentrated to obtain 1.6 g of compound XI-8.

The above XI-8 was dissolved in methanol, added with a 10% KOH aqueous solution, and heated to reflux. TLC showed that XI-8 was reacted completely. The reaction mixture was cooled down to room temperature, added with cyclopentyl methyl ether and layered. The cyclopentyl methyl ether phase was adjusted to pH 6-7 with saturated tartaric acid aqueous solution, concentrated to dryness to remove cyclopentyl methyl ether to obtain 950 mg of V-8 with a yield in two steps of 72.5%.

¹H NMR (DMSO, 400 MHz): 1.60(s, 3H), 1.61 (s, 3H), 1.62-1.71 (m, 2H), 1.91-2.03 (m, 1H), 2.08-2.15 (m, 1H), 3.05(dr, 1H), 3.84-3.88(m, 1H), 4.41 (d, 1H), 4.53 (d, 1H), 5.09 (s, 1H), 5.12 (d, 1H), 5.49 (d, 1H), 6.29 (s, br, 2H), 6.43-6.50 (m, 1H), 8.94 (s, br, 2H). LR-MS (ESI) *m*/*z:* 269 (M-H)⁻.

### Example 15

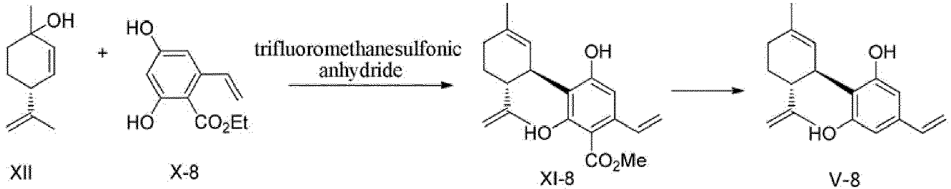

X-8 (1 g, 4.8 mmol) was dissolved in toluene, dropped slowly with trifluoromethanesulfonic anhydride (88mg, 0.1eq) at -10°C to 0°C, followed by dropwise addition of XII (803 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-hexane, xylene, acetonitrile and a 10% KOH aqueous solution and layered. The lower layer was adjusted to pH=6-7 with maleic acid and layered with n-hexane, and the organic phase was concentrated to obtain 1.5 g of XI-8.

The above XI-8 was dissolved in methanol, added with a 10% KOH aqueous solution, and heated to reflux. TLC showed that XI-8 was reacted completely. The reaction mixture was cooled down to room temperature, added with cyclopentyl methyl ether and layered. The cyclopentyl methyl ether phase was adjusted to pH 6-7 with saturated tartaric acid aqueous solution, concentrated to dryness to remove cyclopentyl methyl ether to obtain 850 mg of V-8 with a yield in two steps of 64.9%.

¹H NMR (DMSO, 400 MHz): 1.60(s, 3H), 1.61 (s, 3H), 1.62-1.71 (m, 2H), 1.91-2.03 (m, 1H), 2.08-2.15 (m, 1H), 3.05(dr, 1H), 3.84-3.88(m, 1H), 4.41 (d, 1H), 4.53 (d, 1H), 5.09 (s, 1H), 5.12 (d, 1H),5.49 (d, 1H), 6.29 (s, br, 2H), 6.43-6.50 (m, 1H), 8.94 (s, br, 2H). LR-MS (ESI) *m*/*z:* 269 (M-H)⁻.

### Example 16

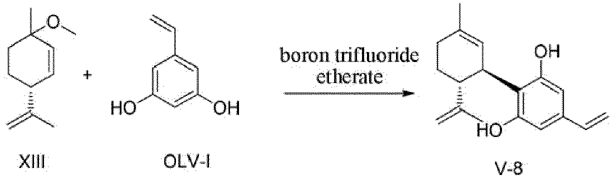

OLV-1 (1.0 g, 7.4 mmol) was dissolved in dichloromethane, added with anhydrous magnesium sulfate, and dropped slowly with a solution of boron trifluoride etherate (0.2 g, 0.1 eq) in toluene at -10°C to 0°C, followed by dropwise addition of XIII (1.35g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, methanol, and a 10% KOH aqueous solution and layered. The organic phase was adjusted to pH=6-7 with saturated tartaric acid aqueous solution, concentrated to dryness to obtain 800mg of V-8 with a yield of 40.2%.

The NMR spectrum of this compound was measured, and it was the same as the product of Example 14.

### Example 17

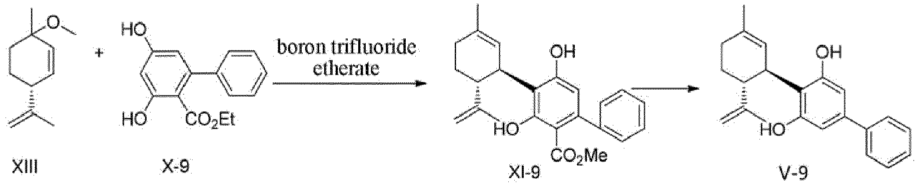

X-9 (1 g, 3.9 mmol) was dissolved in dichloromethane, dropped slowly with boron trifluoride etherate (55 mg, 0.1 eq) at -10°C to 0°C, followed by dropwise addition of XIII (260 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, toluene, methanol and 10% KOH aqueous solution and layered. The lower layer was adjusted to pH=6-7 with maleic acid and layered with cyclohexane, and the organic phase was concentrated to obtain 800mg of XI-9.

The above XI-9 was dissolved in ethanol, added with a 10% NaOH aqueous solution, and heated to reflux. TLC showed that XI-9 was reacted completely. The reaction mixture was cooled down to room temperature, added with cyclopentyl methyl ether and layered. The cyclopentyl methyl ether phase was adjusted to pH=6-7 with saturated tartaric acid aqueous solution, concentrated to dryness to remove cyclopentyl methyl ether to obtain 600 mg of V-9 with a yield in two steps of 62.5%.

¹H NMR (DMSO, 400 MHz): 1.60(s, 3H), 1.64 (s, 3H), 1.64-1.74 (m, 2H), 1.93-2.02 (m, 1H), 2.09-2.18 (m, 1H), 3.11(dr, 1H), 3.91-3.94(m, 1H), 4.46 (d, 1H), 4.56 (d, 1H), 5.14 (s, 1H), 6.48 (s, br, 2H), 7.28-7.33 (m, 1H), 7.40-7.48 (m, 4H), 9.07 (s, br, 2H). LR-MS (ESI) *m*/*z:* 319 (M-H)⁻.

### Example 18

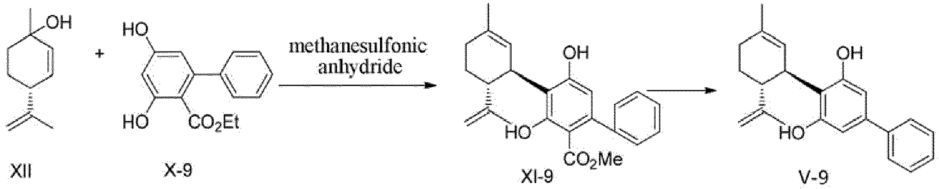

X-9 (1 g, 3.9 mmol) was dissolved in dichloromethane, dropped slowly with methanesulfonic anhydride (100mg, 0.2eq) at -10°C to 0°C, followed by dropwise addition of XII (246mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, toluene, methanol and a 10% KOH aqueous solution and layered. The lower layer was adjusted to pH=6-7 with maleic acid, layered with cyclohexane, and concentrated to obtain 800mg of XI-9.

The above XI-9 was dissolved in ethanol, added with a 10% NaOH aqueous solution, and heated to reflux. TLC showed that XI-9 was reacted completely. The reaction mixture was cooled down to room temperature, added with cyclopentyl methyl ether and layered. The cyclopentyl methyl ether phase was adjusted to pH=6-7 with saturated tartaric acid aqueous solution, concentrated to dryness to remove cyclopentyl methyl ether to obtain 700 mg of V-9 with a yield in two steps of 72.9%.

¹H NMR (DMSO, 400 MHz): 1.60(s, 3H), 1.64 (s, 3H), 1.64-1.74 (m, 2H), 1.93-2.02 (m, 1H), 2.09-2.18 (m, 1H), 3.11(dr, 1H), 3.91-3.94(m, 1H), 4.46 (d, 1H), 4.56 (d, 1H), 5.14 (s, 1H), 6.48 (s, br, 2H), 7.28-7.33 (m, 1H), 7.40-7.48 (m, 4H), 9.07 (s, br, 2H). LR-MS (ESI) *m*/*z:* 319 (M-H)⁻.

### Example 19

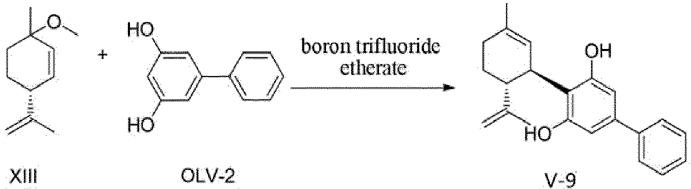

OLV-2 (1.0 g, 5.4 mmol) was dissolved in diethyl ether, added with anhydrous magnesium sulfate, and dropped slowly with a solution of boron trifluoride etherate (0.12 g, 0.1 eq) in diethyl ether at -10°C to 0°C, followed by dropwise addition of XIII (990g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, methanol, and a 10% KOH aqueous solution and layered. The organic phase was adjusted to pH=6-7 with saturated tartaric acid aqueous solution, concentrated to dryness to obtain 800mg of V-9 with a yield of 46.5%.

¹H NMR (DMSO, 400 MHz): 1.60(s, 3H), 1.64 (s, 3H), 1.64-1.74 (m, 2H), 1.93-2.02 (m, 1H), 2.09-2.18 (m, 1H), 3.11(dr, 1H), 3.91-3.94(m, 1H), 4.46 (d, 1H), 4.56 (d, 1H), 5.14 (s, 1H), 6.48 (s, br, 2H), 7.28-7.33 (m, 1H), 7.40-7.48 (m, 4H), 9.07 (s, br, 2H). LR-MS (ESI) *m*/*z:* 319 (M-H)⁻.

### Example 20

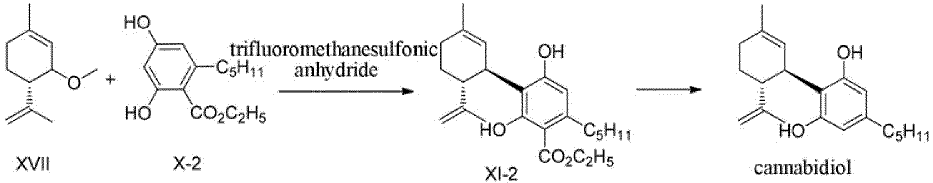

X-2 (1.0 g, 3.76 mmol) was dissolved in chlorobenzene, and dropped slowly with a solution of trifluoromethanesulfonic anhydride (1.0 g, 0.08 eq) in chlorobenzene at -10°C to 0°C, followed by dropwise addition of XVII (681 mg, 1.1eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, chlorobenzene, methanol and a 10% LiOH aqueous solution and layered. The lower layer was adjusted to pH=6-7 with maleic acid, filtered and layered with cyclohexane and the organic phase was concentrated to obtain 1.46g of XI-2.

¹H NMR (DMSO, 400 MHz): 0.87 (t, 3H), 1.24-1.35 (m, 7H), 1.43-1.48 (m, 2H), 1.59 (s, 3H), 1.61 (s, 3H), 1.64-1.72 (m, 2H), 1.92-1.96 (m, 1H), 2.09-2.16 (m, 1H), 2.70 (t, 2H), 3.00-3.06(m, 1H), 3.89-3.92(m, 1H), 4.32 (q, 2H), 4.42(d, 1H), 4.60(d, 1H), 5.09 (s, 1H), 6.20 (s, 1H), 9.92 (s, 1H), 11.80 (s, 1H). LR-MS (ESI) *m*/*z:* 385 (M-H)⁻.

The above XI-2 was dissolved in methanol, added with a 10% LiOH aqueous solution, and heated to reflux. TLC showed that XI-2 was reacted completely. The reaction mixture was added with petroleum ether and layered. The petroleum ether phase was adjusted to pH 6-7 with saturated tartaric acid aqueous solution, concentrated to dryness to remove petroleum ether to obtain 1.02 g of a light brown oil. The above light brown oil was dissolved in n-heptane and stirred at -10°C to 0°C for 4h. A large amount of solids were precipitated out, and filtered to obtain 800 mg of cannabidiol in crystal form A as a white solid, with a yield in two steps of 64.5% and a HPLC purity of 99.5%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H),3.82-3.85(m, 1H),4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 21

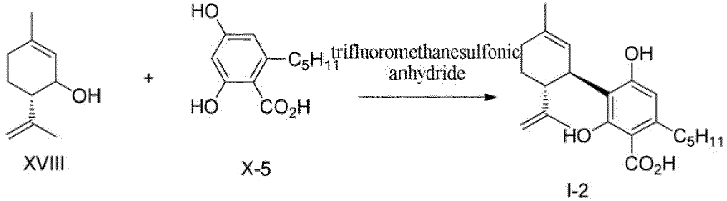

X-5 (1.0 g, 5.6 mmol) was dissolved in dichloromethane/toluene, added with anhydrous magnesium sulfate, and dropped slowly with a solution of trifluoromethanesulfonic anhydride (0.15 g, 0.1 eq) in toluene at -10°C to 0°C, followed by dropwise addition of XVIII (895 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, methanol, and a 10% KOH aqueous solution and layered. The lower layer was adjusted with saturated tartaric acid aqueous solution to pH=5-6, layered with dichloromethane. The organic phase was concentrated to dryness to obtain 800 mg of the product with a total yield of 49.9%.

¹H NMR (CDCl₃, 400 MHz) :0.90 (3H, m), 1.20- 1.33 (4H, m), 1.55 (2H, m), 1.72 (3H s), 1.80 (3H, s), 2.01- 2.39(5H, m), 2.92 (2H, t), 4.10 (1H, m), 4.40 (1H, s), 4.55 (1H, s), 5.57(1H, s), 6.26(1H, s), 6.66(2H, s), 11.85 (1H, s). LR-MS (ESI) *m*/*z:* 357 (M-H)⁻.

### Example 22

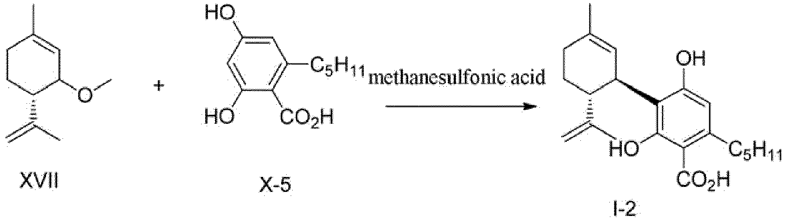

X-5 (1.0 g, 5.6 mmol) was dissolved in chloroform, added with anhydrous magnesium sulfate, and dropped slowly with a solution of methanesulfonic acid (85mg, 0.2 eq) in toluene at -10°C to 0°C, followed by dropwise addition of XVIII (938 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with petroleum ether, methanol, and a 10% KOH aqueous solution and layered. The lower layer was adjusted with saturated tartaric acid aqueous solution to pH=5-6, and layered with n-heptane. The organic phase was concentrated to dryness to obtain 700 mg of the product with a total yield of 43.6%.

¹H NMR (CDCl₃, 400 MHz) :0.90 (3H, m), 1.20- 1.33 (4H, m), 1.55 (2H, m), 1.72 (3H s), 1.80 (3H, s), 2.01- 2.39(5H, m), 2.92 (2H, t), 4.10 (1H, m), 4.40 (1H, s), 4.55 (1H, s), 5.57(1H, s), 6.26(1H, s), 6.66(2H, s), 11.85 (1H, s). LR-MS (ESI) *m*/*z:* 357 (M-H)⁻.

### Example 23

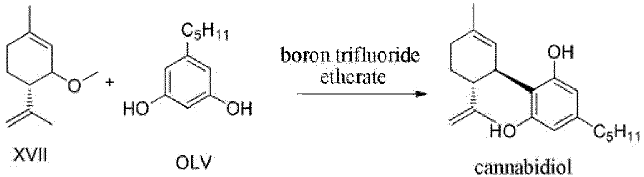

OLV (1.0 g, 5.6 mmol) was dissolved in dichloromethane/toluene, added with anhydrous magnesium sulfate, and dropped slowly with a solution of boron trifluoride etherate (0.15 g, 0.1 eq) in toluene at -10°C to 0°C, followed by dropwise addition of XVII (1.02g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, methanol, and a 10% KOH aqueous solution and layered. The organic phase was adjusted to pH=6-7 with saturated tartaric acid aqueous solution, concentrated to dryness to obtain 800mg of a light brown oil. The above oil was added with petroleum ether, and stirred at -20°C for 12h. A large amount of solids were precipitated out, filtered and dried to obtain 600 mg of the product with a total yield of 34.8%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 24

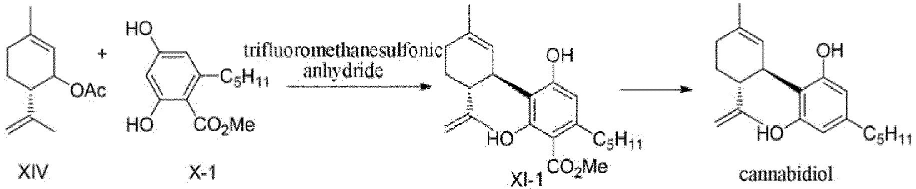

X-1 (1 g, 4.2 mmol) was dissolved in dichloromethane, and dropped slowly with a solution of trifluoromethanesulfonic anhydride (0.1 g, 0.08 eq) in dichloromethane at -10°C to 0°C, followed by dropwise addition of XIV (802 mg, 1.1 eq) at-10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile and a 10% KOH aqueous solution, stirred and layered. The aqueous phase was added with maleic acid to adjust pH=6-7 and layered with dichloromethane, and the organic phase was concentrated to obtain 1.4g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in N,N-dimethylacetamide, added with anhydrous magnesium chloride (800 mg, 2 eq) and N,N-diisopropylethylamine (1.08 g, 2 eq), and heated to 128°C to react overnight. The reaction mixture was cooled down to room temperature, added with methyl tert-butyl ether and layered. The methyl tert-butyl ether phase was adjusted to pH=6-7 with saturated tartaric acid aqueous solution, concentrated to dryness to remove methyl tert-butyl ether to obtain 1.1 g of a light brown oil. The above light brown oil was dissolved in cycloheptane and stirred at -20°C for 12h. A large amount of solids were precipitated out, filtered and dried to obtain 780 mg of cannabidiol in crystal form A with a yield in two steps of 61.2% and a HPLC purity of 99.5%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

The X-ray powder diffraction pattern of the cannabidiol crystal form A was measured, and the result was the same as that in Example 1.

1 g of the white solid above was dissolved in n-heptane under heating, and kept at -30°C to -40°C for 48h. A large amount of crystals were formed, and filtered to obtain 500 mg of single crystal of the crystal form A. The analysis result of the single crystal of the crystal form A is the same as that in Example 1.

### Example 25

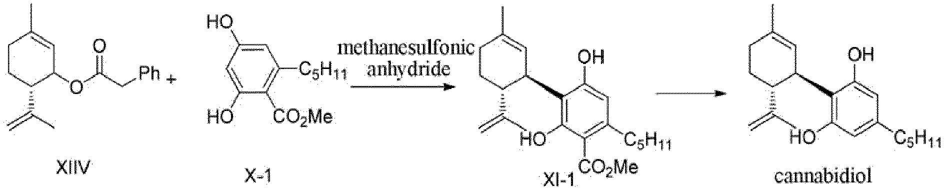

X-1 (1.0 g, 4.2 mmol) was dissolved in dichloromethane, added with anhydrous magnesium sulfate, and dropped slowly with a solution of methanesulfonic anhydride (0.21 g, 0.3 eq) in dichloromethane at -10°C to 0°C, followed by dropwise addition of XIIV (1.24 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with cyclohexane, methyl tert-butyl ether, N,N-dimethylformamide and a 10% LiOH aqueous solution and layered. The lower layer was adjusted to pH=6-7 with fumaric acid and layered with ethyl acetate, and the organic phase was concentrated to obtain 1.12 g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in ethanol, added with a 10% NaOH aqueous solution, and heated to reflux. TLC showed that XI-1 was reacted completely. The reaction mixture was added with diethyl ether and layered. The diethyl ether phase was adjusted to pH 6-7 with saturated tartaric acid aqueous solution, concentrated to dryness to remove diethyl ether to obtain 0.85 g of a light brown oil. The above light brown oil was added with cyclohexane and stirred at -20°C for 12h. A large amount of solids were precipitated out, filtered and dried to obtain 680 mg of cannabidiol in crystal form A with a yield in two steps of 53.0%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 26

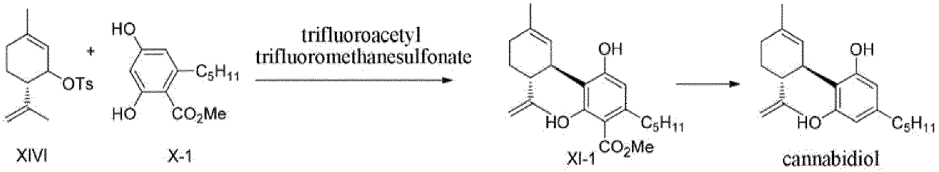

X-1 (1.0 g, 4.2 mmol) was dissolved in tetrahydrofuran, dropped slowly a solution of trifluoroacetyl trifluoromethanesulfonate (0.11 g, 0.1 eq) in tetrahydrofuran at -10°C to 0°C, followed by dropwise addition of XIVI (1.42 g, 1.1 eq). TLC showed that the reaction was almost complete. The reaction mixture was added with saturated sodium bicarbonate to quench the reaction, added with water and layered. The organic phase was concentrated to a small volume, added with n-hexane, cyclopentyl methyl ether, methanol and a 10% KOH aqueous solution and layered. The lower layer was adjusted with tartaric acid to pH=6-7 and layered with isopropyl acetate, and the organic phase was concentrated to obtain 1.28 g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in N,N-dimethylacetamide, added with anhydrous magnesium chloride (800 mg, 2 eq) and N,N-diisopropylethylamine (1.08 g, 2 eq), and heated to 128°C to react overnight. The reaction mixture was cooled down to room temperature, added with toluene and layered. The toluene phase was adjusted to pH=6-7 with saturated tartaric acid aqueous solution, concentrated to dryness to remove toluene to obtain 0.81 g of a light brown oil. The above light brown oil was added with n-hexane and stirred at -20°C for 12h. A large amount of solids were precipitated out, filtered and dried to obtain 650 mg of cannabidiol in crystal form A with a yield in two steps of 50.4%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 27

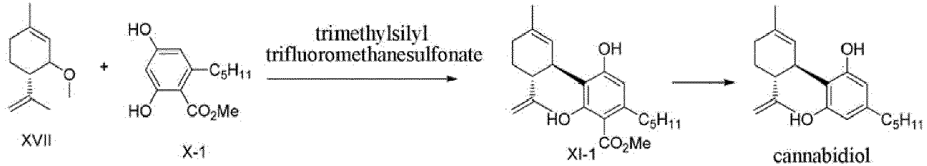

X-1 (1.0 g, 4.2 mmol) was dissolved in n-heptane, dropped slowly with a solution of trimethylsilyl trifluoromethanesulfonate (0.1 g, 0.1 eq) in toluene at -10°C to 0°C, followed by dropwise addition of XVII (702 mg, 1.leq) at -10°C to 0°C. TLC showed that the reaction was almost complete. The reaction mixture was added with saturated sodium bicarbonate to quench the reaction, added with water and layered. The organic phase was concentrated to a small volume, added with xylene, n-heptane, dimethyl sulfoxide and a 10% LiOH aqueous solution and layered. The aqueous phase was adjusted to pH=6-7 with citric acid and layered with chloroform, and the organic phase was concentrated to obtain 1.44g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in ethylene glycol, and added with a 10% LiOH aqueous solution. The resulting mixture was heated to reflux. TLC showed that XI-1 was reacted completely. The reaction mixture was added with isopropyl ether and layered. The isopropyl ether phase was adjusted to pH=6-7 with saturated tartaric acid aqueous solution, concentrated to dryness to remove isopropyl ether to obtain 0.9 g of a light brown oil. The above light brown oil was added with cyclohexane and stirred at -20°C for 12h. A large amount of solids were precipitated out, filtered and dried to obtain 750 mg of cannabidiol in crystal form A with a yield in two steps of 58.1%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 28

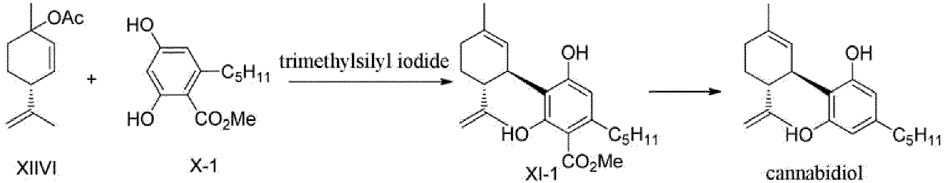

X-1 (1.0 g, 4.2 mmol) was dissolved in methyl tert-butyl ether, added with sodium sulfate, and dropped slowly with a solution of trimethylsilyl iodide (0.84 g, 0.1 eq) in methyl tert-butyl ether at -10°C to 0°C, followed by dropwise addition of XIIVI (900 mg, 1.1 eq) at -10°C to 0°C. TLC showed that the reaction was almost complete. The reaction mixture was added with saturated sodium bicarbonate to quench the reaction, and added with water and layered. The organic phase was concentrated to a small volume, added with cyclohexane, N,N-methylacetamide and a 10% NaOH aqueous solution and layered. The lower layer was adjusted to pH=6-7 with maleic acid and layered with cyclohexane, and the organic phase was concentrated to obtain 1.39 g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H),4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in acetonitrile, added with a 10% LiOH aqueous solution, and heated to reflux. TLC showed that XI-1 was reacted completely. The reaction mixture was added with n-hexane and layered. The n-hexane phase was adjusted to pH 6-7 with saturated tartaric acid aqueous solution, concentrated to dryness to remove n-hexane to obtain 0.9 g of a light brown oil. The above light brown oil was added with n-hexane and stirred at -20°C for 12h. A large amount of solids were precipitated out, filtered and dried to obtain 750 mg of cannabidiol in crystal form A with a yield in two steps of 58.0%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 29

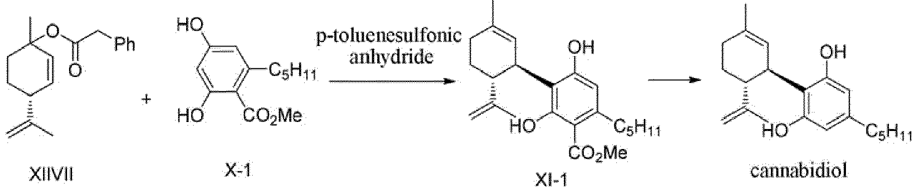

X-1 (1.0g, 4.2mmol) was dissolved in 1,2-dichloroethane, dropped slowly with a solution of p-toluenesulfonic anhydride (1.36g, 0.1eq) in 1,2-dichloroethane at -10°C to 0°C, followed by dropwise addition of XIIVII (1.24 mg, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with petroleum ether, methanol and a 10% KOH aqueous solution and layered. The lower layer was adjusted to pH=6-7 with maleic acid, filtered and layered with n-octane, and the organic phase was concentrated to obtain 1.32 g of XI-1 as an oil.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m, 1H), 3.84(s, 3H), 3.89-3.92(m, 1H), 4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in toluene, added with a 10% LiOH aqueous solution, and heated to reflux. TLC showed that XI-1 was reacted completely. The reaction mixture was added with n-octane and layered. The n-octane phase was adjusted to pH 6-7 with saturated tartaric acid aqueous solution, concentrated to dryness to remove n-octane to obtain 0.9 g of a light brown oil. The above light brown oil was added with n-octane and stirred at -20°C for 12h. A large amount of solids were precipitated out, filtered and dried to obtain 680 mg of cannabidiol in crystal form A with a yield in two steps of 52.7%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 30

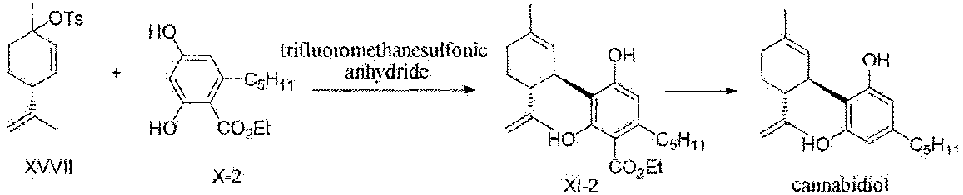

X-2 (1.0 g, 3.76 mmol) was dissolved in chlorobenzene, dropped slowly with a solution of trifluoromethanesulfonic anhydride (1.0 g, 0.08 eq) in chlorobenzene at -10°C to 0°C, followed by dropwise addition of XVVII (1.42 g, 1.1 eq) at -10°C to 0°C. TLC showed that the reaction was almost complete. The reaction mixture was added with saturated sodium bicarbonate to quench the reaction, added with water and layered. The organic phase was concentrated to a small volume, added with n-heptane, xylene, methanol and a 10% LiOH aqueous solution and layered. The lower layer was adjusted to pH=6-7 with maleic acid, filtered and layered with n-heptane, and the organic phase was concentrated to obtain 1.36g of XI-2.

¹H NMR (DMSO, 400 MHz): 0.87 (t, 3H), 1.24-1.35 (m, 7H), 1.43-1.48 (m, 2H), 1.59 (s, 3H), 1.61 (s, 3H), 1.64-1.72 (m, 2H), 1.92-1.96 (m, 1H), 2.09-2.16 (m, 1H), 2.70 (t, 2H), 3.00-3.06(m, 1H), 3.89-3.92(m, 1H), 4.32 (q, 2H), 4.42(d, 1H), 4.60(d, 1H), 5.09 (s, 1H), 6.20 (s, 1H), 9.92 (s, 1H), 11.80 (s, 1H). LR-MS (ESI) *m*/*z:* 385 (M-H)⁻.

The above XI-2 was dissolved in methanol, added with a 10% LiOH aqueous solution, and heated to reflux. TLC showed that XI-2 was reacted completely. The reaction mixture was added with petroleum ether and layered. The petroleum ether phase was adjusted to pH 6-7 with saturated tartaric acid aqueous solution, concentrated to dryness to remove petroleum ether to obtain 0.9 g of a light brown oil. The above light brown oil was dissolved in n-heptane and stirred at -10°C to 0°C for 4h. A large amount of solids were precipitated out, and filtered to obtain 850 mg of a white solid. The above solid was added with cyclopentane and stirred at -20°C for 12h. A large amount of solids were precipitated out, filtered and dried to obtain 850 mg of cannabidiol in crystal form A with a yield in two steps of 65.8%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 31

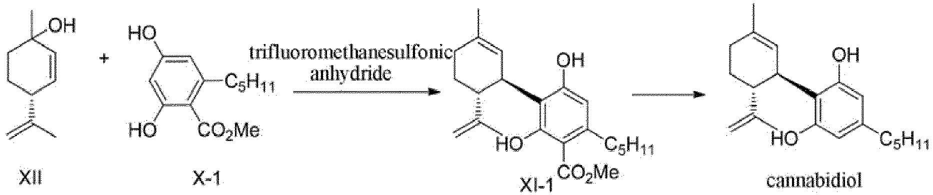

X-1 (1 g, 4.2 mmol) was dissolved in dichloromethane, added with a solution of trifluoromethanesulfonic anhydride (0.1 g, 0.08 eq) in dichloromethane at -10°C to 0°C, followed by dropwise addition of XII (702 mg, 1.1 eq) at-10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile and a 10% KOH aqueous solution, stirred and layered. The aqueous phase was added with maleic acid to adjust pH=6-7, layered with dichloromethane, and concentrated to obtain 1.3g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m,1H),3.84(s,3H), 3.89-3.92(m,1H),4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in dimethyl sulfoxide, added with magnesium tert-butoxide (1.4 g, 2 eq), and heated at 120-130 °C. TLC showed that XI-1 was reacted completely. The reaction mixture was added with methyl tert-butyl ether and layered. The methyl tert-butyl ether phase was adjusted to pH=6-7 with saturated tartaric acid aqueous solution, concentrated to dryness to remove methyl tert-butyl ether to obtain 0.9 g of a light brown oil. The above light brown oil was dissolved in cycloheptane and stirred at -20°C for 12h. A large amount of solids were precipitated out, filtered and dried to obtain 680 mg of cannabidiol in crystal form A with a yield in two steps of 53.4% and a HPLC purity of 99.5%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr, 1H), 3.82-3.85(m, 1H), 4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 32

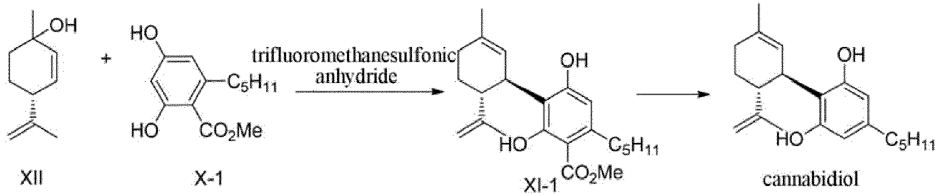

X-1 (1 g, 4.2 mmol) was dissolved in dichloromethane, added with a solution of trifluoromethanesulfonic anhydride (0.1 g, 0.08 eq) in dichloromethane at -10°C to 0°C, followed by dropwise addition of XII (702 mg, 1.1 eq) at-10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile and a 10% KOH aqueous solution, stirred and layered. The aqueous phase was added with maleic acid to adjust pH=6-7 and layered with dichloromethane, and the organic phase was concentrated to obtain 1.3g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m,1H),3.84(s,3H), 3.89-3.92(m,1H),4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in dimethyl sulfoxide, added with magnesium isopropoxide (1.2 g, 2 eq), and heated at 120-130 °C. TLC showed that XI-1 was reacted completely. The reaction mixture was added with methyl tert-butyl ether and layered. The methyl tert-butyl ether phase was adjusted to pH=6-7 with saturated tartaric acid aqueous solution, concentrated to dryness to remove methyl tert-butyl ether to obtain 0.9 g of a light brown oil. The above light brown oil was dissolved in cycloheptane and stirred at -20°C for 12h. A large amount of solids were precipitated out, filtered and dried to obtain 680 mg of cannabidiol in crystal form A with a yield in two steps of 53.4% and a HPLC purity of 99.5%.

¹H NMR (DMSO, 400 MHz): 0.87 (t, 3H), 1.20-1.33 (m, 4H), 1.44-1.51 (m, 2H), 1.59 (s, 3H), 1.60 (s, 3H), 1.63-1.70 (m, 2H), 1.89-1.94 (m, 1H), 2.07-2.13 (m, 1H), 2.30 (t, 2H), 3.04(dr,1H),3.82-3.85(m,1H),4.41 (d, 1H), 4.50 (d, 1H), 5.09 (s, 1H), 6.02 (s, br, 2H), 8.60 (s, br, 2H). LR-MS (ESI) *m*/*z:* 313 (M-H)⁻.

### Example 33

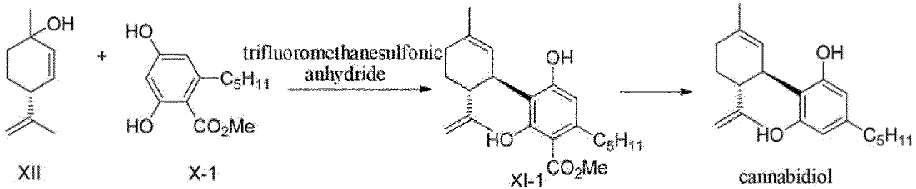

X-1 (1 g, 4.2 mmol) was dissolved in dichloromethane, added with a solution of trifluoromethanesulfonic anhydride (0.1 g, 0.08 eq) in dichloromethane at -10°C to 0°C, followed by dropwise addition of XII (702 mg, 1.1 eq) at-10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile and a 10% KOH aqueous solution, stirred and layered. The aqueous phase was added with maleic acid to adjust pH=6-7 and layered with dichloromethane, and the organic phase was concentrated to obtain 1.3g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m,1H),3.84(s,3H), 3.89-3.92(m,1H),4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in N,N-dimethylacetamide, added with anhydrous magnesium chloride (800 mg, 2 eq) and sodium tert-butoxide (860 mg, 2 eq), and heated to 128°C to react overnight. The reaction mixture was cooled down to room temperature, added with methyl tert-butyl ether and layered. The methyl tert-butyl ether phase was adjusted to pH=6-7 with saturated tartaric acid aqueous solution, concentrated to dryness to remove methyl tert-butyl ether to obtain 1.1 g of a light brown oil. The above light brown oil was dissolved in cycloheptane and stirred at -20°C for 12h. A large amount of solids were precipitated out, filtered and dried to obtain 740 mg of cannabidiol in crystal form A with a yield in two steps of 58.1% and a HPLC purity of 99.5%.

### Example 34

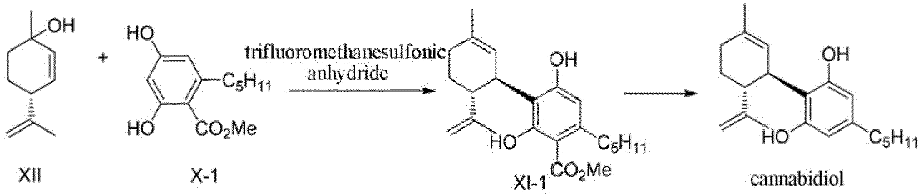

X-1 (1 g, 4.2 mmol) was dissolved in dichloromethane, added with a solution of trifluoromethanesulfonic anhydride (0.1 g, 0.08 eq) in dichloromethane at -10°C to 0°C, followed by dropwise addition of XII (702 mg, 1.1 eq) at-10°C to 0°C. TLC showed that the reaction was almost complete. The reaction mixture was added with saturated sodium bicarbonate to quench the reaction, added with water and layered. The organic phase was concentrated to a small volume, added with n-heptane, toluene, acetonitrile and a 10% KOH aqueous solution, stirred and layered. The aqueous phase was added with maleic acid to adjust pH=6-7 and layered with dichloromethane, and the organic phase was concentrated to obtain 1.3g of XI-1.

¹H NMR (DMSO, 400 MHz): 0.87 (t, 3H), 1.24-1.33 (m, 4H), 1.41-1.48 (m, 2H), 1.59 (s, 3H), 1.61 (s, 3H), 1.64-1.72 (m, 2H), 1.92-1.96 (m, 1H), 2.09-2.13 (m, 1H), 2.68 (t, 2H), 3.00-3.06(m,1H),3.84(s,3H), 3.89-3.92(m,1H),4.44(d, 2H), 5.09 (s, 1H), 6.21 (s, 1H), 9.90 (s, 1H), 11.56 (s, 1H). LR-MS (ESI) *m*/*z:* 371 (M-H)⁻.

The above XI-1 was dissolved in dimethyl sulfoxide, added with anhydrous magnesium chloride (800 mg, 2 eq) and sodium hydroxide (336 mg, 2 eq), and heated to 128°C to react overnight. The reaction mixture was cooled down to room temperature, added with methyl tert-butyl ether and layered. The methyl tert-butyl ether phase was adjusted to pH=6-7 with saturated tartaric acid aqueous solution, concentrated to dryness to remove methyl tert-butyl ether to obtain 1.1 g of a light brown oil. The above light brown oil was dissolved in cycloheptane and stirred at -20°C for 12h. A large amount of solids were precipitated out, filtered and dried to obtain 680 mg of cannabidiol in crystal form A with a yield in two steps of 53.3% and a HPLC purity of 99.5%.

The above examples are only to illustrate the technical concept and characteristics of the present invention, so that a person skilled in the art can understand the content of the present invention and implement them accordingly, and the protection scope of the present invention is not limited by them. All equivalent changes or modifications made according to the spirit and scope of the present invention should be covered by the protection scope of the present invention.

## Claims

1. A method for preparing a compound of formula I, which is one of the following methods: 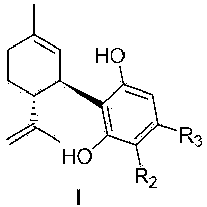 Method One:
reacting a compound of formula II with a compound of formula III in the presence of a catalyst M to produce the compound of formula I, 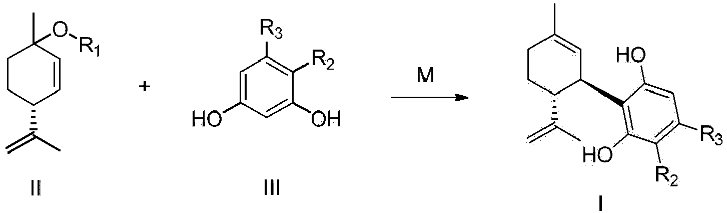 Method Two:
reacting a compound of formula VI with a compound of formula III in the presence of a catalyst M to produce the compound of formula I, 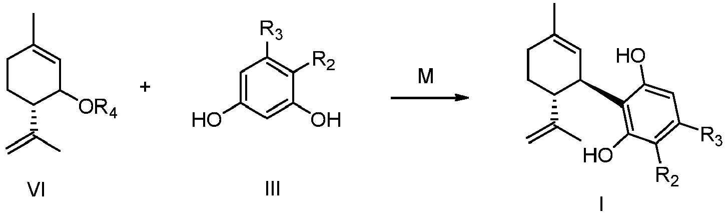 in formulas I, II, III and VI,
R₁ and R₄ are each independently hydrogen, -(O=)CR₁ₐ, -SO₂R_{1b}, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; wherein R₁ₐ and R_{1b} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl, preferably, R₁ₐ and R_{1b} are each independently hydrogen, methyl, ethyl, propyl, phenyl, benzyl, phenethyl or phenylpropyl, wherein the substituent for the "substituted" is hydroxyl, amino, mercapto, an organophosphorus group, halogen, C₁-C₁₀ alkyl, C₃-C₁₀ cycloalkyl, or C₁-C₁₀ alkoxy,
further preferably, R₁ and R₄ are each independently hydrogen, methyl, ethyl, cyclopropylmethyl, methoxymethyl, 2-methoxyethyl, acetyl, propionyl, benzoyl, phenylacetyl, phenylpropionyl, methanesulfonyl, trifluoromethanesulfonyl or p-toluenesulfonyl,
R₂ is hydrogen, carboxyl, -COOR₅ or -CONR^{c}R^{d}; wherein R₅ is C₁-C₂₀ linear or branched alkyl, C₃-C₂₀ cycloalkyl, benzyl, or C₆-C₂₀ aryl; preferably, R₅ is methyl or ethyl; wherein R^{c} and R^{d} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; the substituent for the "substituted" is hydroxyl, amino, mercapto, an organophosphorus group, halogen, C₁-C₁₀ alkyl, C₃-C₁₀ cycloalkyl, or C₁-C₁₀ alkoxy; preferably, R^{c} and R^{d} are each independently hydrogen, methyl, ethyl or hydroxyethyl;
further preferably, R₂ is hydrogen, -COOH, -COOCH₃, -COOC₂H₅ or -(CH₂CH₂)₂NCH₃,
R₃ is substituted or unsubstituted C₁-C₂₀ linear or branched alkyl, substituted or unsubstituted C₃-C₂₀ cycloalkyl, substituted or unsubstituted C₃-C₂₀ alkenyl, substituted or unsubstituted C₃-C₂₀ alkynyl, substituted or unsubstituted C₃-C₂₀ acyl, substituted or unsubstituted C₆-C₂₀ aryl, or substituted or unsubstituted C₂-C₂₀ heteroaryl containing one or more atoms selected from the group consisting of oxygen, nitrogen, sulfur and phosphorus, wherein the substituent for the "substituted" is one or more selected from the group consisting of halogen; hydroxyl; C₁-C₂₀ alkyl; -O-C₁-C₂₀ alkyl; -NR^{a}R^{b}, wherein R^{a} and R^{b} are each independently selected from the group consisting of hydrogen and C₁-C₄ alkyl; -SO-C₁-C₂₀ alkyl; -SO₂-C₁-C₂₀ alkyl; C₃-C₂₀ alkenyl; C₃-C₂₀ alkynyl; C₁-C₂₀ acyl; C₆-C₂₀ aryl; C₂-C₂₀ heteroaryl containing one or more atoms selected from the group consisting of oxygen, nitrogen, sulfur and phosphorus; C₃-C₂₀ cycloalkyl; and C₂-C₂₀ heterocyclyl containing one or more atoms selected from the group consisting of oxygen, nitrogen, sulfur and phosphorus; preferably, R₃ is -C₅H₁₁.

2. The method according to claim 1, wherein,
R₁ and R₄ are each independently -(O=)CR₁ₐ, -SO₂R_{1b}, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl,
the catalyst M is one or more selected from the group consisting of Lewis acids, protic acids, acid anhydrides, silylesters, and silanes;
preferably,
the Lewis acid is selected from the group consisting of boron trihalides, more preferably, boron trichloride, boron trifluoride; aluminum trihalides, more preferably, aluminum trichloride, aluminum tribromide; transition metal salts, especially transition metal halide or trifluoromethanesulfonate, more preferably titanium tetrachloride, zinc chloride, zinc bromide, zinc trifluoromethanesulfonate; halides of the elements of the fourth, fifth, and sixth main groups in the periodic table of elements, more preferably, tin tetrachloride, phosphorus oxychloride, thionyl chloride, sulfone dichloride;
the protic acid is selected from the group consisting of perchloric acid, hydrogen halides, sulfuric acid, bisulfates, phosphoric acid, biphosphates, pyrophosphoric acid, R₆COOH and R₇SO₃H; wherein R₆ and R₇ are each independently substituted or unsubstituted C₁-C₃₀ linear or branched alkyl, substituted or unsubstituted C₃-C₃₀ cycloalkyl, substituted or unsubstituted benzyl, or substituted or unsubstituted C₆-C₃₀ aryl, wherein the substituent for the "substituted" is one or more selected from the group consisting of carboxyl, phosphoric acid group, sulfonic acid group, phosphorous acid group and halogen;
the acid anhydride is one or more selected from the group consisting of trifluoromethanesulfonic anhydride, trifluoroacetic anhydride, methanesulfonic anhydride, ethanesulfonic anhydride, phenylmethanesulfonic anhydride, p-toluenesulfonic anhydride, trifluoroacetyl trifluoromethanesulfonate;
the silylester is one or two selected from the group consisting of trimethylsilyl trifluoromethanesulfonate and triethylsilyl trifluoromethanesulfonate;
the silane is one or more selected from the group consisting of trimethyliodosilane, triethyliodosilane, trimethylbromosilane, and trimethylchlorosilane.

3. The method according to claim 1, wherein R₁ and R₄ are each independently hydrogen, and the catalyst M is one or more selected from the group consisting of acid anhydrides, silylesters, and silanes;
preferably,
the acid anhydride is one or more selected from the group consisting of trifluoromethanesulfonic anhydride, trifluoroacetic anhydride, methanesulfonic anhydride, ethanesulfonic anhydride, phenylmethanesulfonic anhydride, and p-toluenesulfonic anhydride;
the silylester is trimethylsilyl trifluoromethanesulfonate;
the silane is one or more selected from the group consisting of trimethyliodosilane, triethyliodosilane, trimethylbromosilane and trimethylchlorosilane;
more preferably, the catalyst M is one or more selected from the group consisting of methanesulfonic anhydride, trifluoromethanesulfonic anhydride, trimethylsilyl trifluoromethanesulfonate and trimethylsilyl iodide.

4. The method according to claim 1, wherein the feeding molar ratio of the catalyst M to the compound of formula II or VI is 0.01:1 to 1:1, preferably 0.03:1 to 0.5:1, and more preferably 0.05 :1 to 0.5:1.

5. The method according to claim 1, wherein the compound of formula II or VI is reacted with the compound of formula III in a solvent, the solvent is one or a mixture of two or more selected from the group consisting of alkanes, aromatic hydrocarbons, halogenated hydrocarbons, esters, ethers and polar aprotic solvents,
preferably,
the alkane is a C₅-C₂₀ linear or branched or cyclic alkane;
the aromatic hydrocarbon is a substituted benzene compound;
the halogenated hydrocarbon is selected from the group consisting of dichloromethane, 1,2-dichloroethane and chloroform;
the ester is selected from the group consisting of methyl acetate, ethyl acetate, isopropyl acetate and butyl acetate;
the ether is selected from the group consisting of tetrahydrofuran, dioxane, 2-methyltetrahydrofuran, diethyl ether, ethylene glycol dimethyl ether, cyclopentyl methyl ether and methyl tert-butyl ether;
the polar aprotic solvent is selected from the group consisting of acetonitrile, acetone, N,N-dimethylformamide, dimethylsulfoxide, N-methylpyrrolidone and dimethylsulfoxide,
further preferably, the solvent is one or a mixture of two or more selected from the group consisting of n-heptane, n-hexane, toluene, xylene, chlorobenzene, dichloromethane, ethyl acetate, and tetrahydrofuran.

6. The method according to claim 1, wherein the feeding molar ratio of the compound of formula II or VI to the compound of formula III is 1:5 to 5:1, preferably 0.5:1 to 2:1, and more preferably 0.8: 1 to 1.5:1.

7. The method according to claim 1, wherein:
the reaction is carried out at a temperature of -30°C to 50°C, preferably -20°C to 40°C; and/or
the reaction time of the reaction is 1h to 24h.

8. The method according to claim 1, further comprising a step of purifying the compound of formula I by layering, preferably, the step of purifying the compound of formula I includes two routes:
Route (a):
in the case that the reaction solvent is a polar aprotic solvent selected from the group consisting of acetonitrile, acetone, N,N-dimethylformamide, dimethylsulfoxide, N-methylpyrrolidone and dimethylsulfoxide, after the reaction between the compound of formula II or VI and the compound of formula III is completed, the reaction mixture is added with saturated sodium bicarbonate to quench the reaction, added with an aqueous inorganic alkali solution and then with a solvent or a mixed solvent of two or more selected from the group consisting of hydrocarbon solvents and ether solvents for layering;
Route (b):
in the case that the reaction solvent is one or a mixture of two or more selected from the group consisting of alkanes, aromatic hydrocarbons, halogenated hydrocarbons, esters and ethers other than the polar aprotic solvent, after the reaction between the compound of formula II or VI and the compound of formula III is completed, the reaction mixture is added with saturated sodium bicarbonate to quench the reaction, and then with water and layered, and the organic phase is concentrated to remove the solvent to obtain a crude product of formula I, which is added with an aqueous inorganic alkali solution, and then with a solvent or a mixed solvent of two or more selected from the group consisting of hydrocarbon solvents and ether solvents for layering.

9. The method according to claim 8, wherein the solvent used for layering is a mixture of one or a mixed solvent of two or more selected from the group consisting of hydrocarbon solvents and ether solvents, and an aqueous inorganic alkali solution;
the hydrocarbon solvent includes alkane solvents selected from the group consisting of C₅-C₂₀ linear or branched or cyclic alkane; and unsaturated aromatic hydrocarbon solvents selected from the group consisting of toluene, chlorobenzene, xylene and nitrobenzene;
the ether solvent is selected from the group consisting of tetrahydrofuran, methyl tert-butyl ether, diethyl ether, cyclopentyl methyl ether, isopropyl ether, anisole, ethylene glycol dimethyl ether, tetrahydropyran, and dioxane;
the inorganic alkali in the aqueous inorganic alkali solution is one or a mixture of two or more selected from sodium hydroxide, potassium hydroxide, lithium hydroxide, sodium carbonate, potassium carbonate, and lithium carbonate, the mass fraction of the aqueous inorganic alkali solution is 1%-30%;
preferably, the step of purifying the compound of formula I further comprises: adjusting the pH of the aqueous phase to 6-7 with an acid after layering, adding an organic solvent to the aqueous phase for extraction, and concentrating the combined organic phase to dryness to obtain the compound of formula I,
wherein, preferably, the acid used may be one of organic acids and inorganic acids, wherein the organic acid used is selected from the group consisting of formic acid, acetic acid, propionic acid, oxalic acid, maleic acid, citric acid, tartaric acid, methanesulfonic acid, benzenesulfonic acid, wherein the inorganic acid used is selected from the group consisting of hydrochloric acid, sulfuric acid, and phosphoric acid;
wherein, preferably, the organic solvent is one or a mixture of two or more selected from the group consisting of 2-methyltetrahydrofuran, diethyl ether, ethylene glycol dimethyl ether, a C₅-C₂₀ linear or branched or cyclic alkane, benzene, toluene, xylene, dichloromethane, 1,2-dichloroethane, chloroform, cyclopentyl methyl ether, and methyl tert-butyl ether, preferably selected from the group consisting of dichloromethane, tetrahydrofuran, toluene, xylene, chlorobenzene, n-heptane, and n-hexane.

10. A method for preparing a compound of formula V, comprising:
using the method of any one of claims 1-9 to prepare the compound of formula I,
removing R2 from the compound of formula I to produce a compound of formula V, 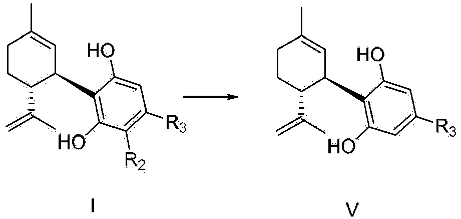
wherein, R₂ is carboxyl, -COOR₅ or -CONR^{c}R^{d}; wherein R₅ is C₁-C₂₀ linear or branched alkyl, C₃-C₂₀ cycloalkyl, benzyl, or C₆-C₂₀ aryl; preferably, R₅ is methyl or ethyl; and wherein R^{c} and R^{d} are each independently hydrogen, substituted or unsubstituted C₁-C₂₀ linear or branched alkyl, substituted or unsubstituted C₃-C₂₀ cycloalkyl, substituted or unsubstituted benzyl, or substituted or unsubstituted C₆-C₂₀ aryl; the substituent for the "substituted" is hydroxyl, amino, mercapto, an organophosphorus group, halogen, C₁-C₁₀ alkyl, C₃-C₁₀ cycloalkyl, or C₁-C₁₀ alkoxy; preferably, R^{c} and R^{d} are each independently hydrogen, methyl, ethyl or hydroxyethyl;
further preferably, R₂ is hydrogen, -COOH, -COOCH₃, -COOC₂H₅ or -(CH₂CH₂)₂NCH₃,
R₃ is substituted or unsubstituted C₁-C₂₀ linear or branched alkyl, substituted or unsubstituted C₃-C₂₀ cycloalkyl, substituted or unsubstituted C₃-C₂₀ alkenyl, substituted or unsubstituted C₃-C₂₀ alkynyl, substituted or unsubstituted C₃-C₂₀ acyl, substituted or unsubstituted C₆-C₂₀ aryl, or substituted or unsubstituted C₂-C₂₀ heteroaryl containing one or more atoms selected from the group consisting of oxygen, nitrogen, sulfur and phosphorus, wherein the substituent for the "substituted" is one or more selected from the group consisting of halogen; hydroxyl; C₁-C₂₀ alkyl; -O-C₁-C₂₀ alkyl; -NR^{a}R^{b}, wherein R^{a} and R^{b} are each independently selected from the group consisting of hydrogen and C₁-C₄ alkyl; -SO-C₁-C₂₀ alkyl; -SO₂-C₁-C₂₀ alkyl; C₃-C₂₀ alkenyl; C₃-C₂₀ alkynyl; C₁-C₂₀ acyl; C₆-C₂₀ aryl; C₂-C₂₀ heteroaryl containing one or more atoms selected from the group consisting of oxygen, nitrogen, sulfur and phosphorus; C₃-C₂₀ cycloalkyl; and C₂-C₂₀ heterocyclyl containing one or more atoms selected from the group consisting of oxygen, nitrogen, sulfur and phosphorus; preferably, R₃ is -C₅H₁₁.

11. The method according to claim 10, wherein the reaction of removing R2 from the compound of formula I to produce the compound of formula V is carried out in the presence of one or a mixture of two or more selected from the group consisting of alkalis, and an alkali metal or alkaline earth metal salts;
wherein preferably, the alkali is one or a mixture of two or more selected from the group consisting of alkali metal or alkaline earth metal hydroxides, alkali metal or alkaline earth metal carbonates, alkali metal or alkaline earth metal alkoxides, and nitrogen-containing organic bases, more preferably selected from the group consisting of sodium hydroxide, potassium hydroxide, lithium hydroxide, sodium carbonate, potassium carbonate, lithium carbonate, sodium methoxide, potassium methoxide, lithium ethoxide, sodium ethoxide, magnesium methoxide, magnesium ethoxide, magnesium n-propoxide, calcium methoxide, calcium ethoxide, sodium tert-butoxide, lithium tert-butoxide, magnesium tert-butoxide, magnesium isobutoxide, magnesium tert-amyloxide, N-methylmorpholine, N,N-diisopropylethylamine, triethylamine, tripropylamine, tri-n-propylamine, triisopropylamine, tributylamine, pyridine, pyrimidine, quinoline, N-methylpiperidine, N-methylpiperazine, imidazole, dimethylaminopyridine, N-methylmorpholine, dimethylaniline, 1,8-diazabicyclo[5.4.0]undec-7-ene (DBU), 1,5-diazabicyclo[4.3.0]non-5-ene (DBN), and 1,4-diazabicyclo[2.2.2]octane (DABCO); preferably, the alkali is selected from the group consisting of sodium hydroxide, potassium hydroxide, lithium hydroxide, sodium carbonate, potassium carbonate, lithium carbonate, magnesium ethoxide, magnesium n-propoxide, magnesium tert-butoxide and magnesium isobutoxide; more preferably, the alkali is sodium hydroxide, potassium hydroxide, lithium hydroxide, sodium carbonate, potassium carbonate, lithium carbonate, magnesium ethoxide, or magnesium tert-butoxide;
wherein, preferably, the alkali metal or alkaline earth metal salt is one or a mixture of two or more selected from the group consisting of alkali metal or alkaline earth metal halides, alkali metal or alkaline earth metal carboxylates, and alkali metal or alkaline earth metal sulfonates; more preferably the alkali metal or alkaline earth metal salt is selected from the group consisting of lithium chloride, lithium bromide, lithium iodide, lithium acetate, lithium trifluoroacetate, lithium benzoate, lithium trifluoromethanesulfonate, lithium methanesulfonate, lithium phenylmethanesulfonate; sodium chloride, sodium bromide, sodium iodide, sodium acetate, sodium trifluoroacetate, sodium benzoate, sodium trifluoromethanesulfonate, sodium methanesulfonate, sodium phenylmethanesulfonate; potassium chloride, potassium bromide, potassium iodide, potassium acetate, potassium trifluoroacetate, potassium benzoate, potassium trifluoromethanesulfonate, potassium methanesulfonate, potassium phenylmethanesulfonate; magnesium chloride, magnesium bromide, magnesium iodide, magnesium acetate, magnesium trifluoroacetate, benzoic acid magnesium, magnesium trifluoromethanesulfonate, magnesium methanesulfonate, magnesium phenylmethanesulfonate, calcium chloride, calcium bromide, calcium acetate, calcium trifluoroacetate, and calcium benzoate; preferably, the alkali metal or alkaline earth metal salt is lithium bromide, lithium chloride, sodium chloride, potassium chloride, magnesium bromide, or magnesium chloride; more preferably, the alkali metal or alkaline earth metal salt is magnesium chloride or lithium chloride;
wherein, a solvent is used to carry out the reaction, preferably, the solvent is one or a mixture of two or more selected from the group consisting of methanol, ethanol, isopropanol, ethylene glycol, tetrahydrofuran, dioxane, 2-methyltetrahydrofuran, diethyl ether, ethylene glycol dimethyl ether, cyclopentyl methyl ether, methyl tert-butyl ether, dimethyl sulfoxide, N-methylpyrrolidone, N,N-dimethylformamide, N,N-dimethylacetamide, N,N-diethylformamide, N,N-diethylacetamide and water.

12. The method according to claim 11, wherein the feeding molar ratio of the alkali metal or alkaline earth metal salt to the compound of formula I is 20:1 to 1:4, preferably 15:1 to 1:2, more preferably 10:1 to 1:1; and the feeding molar ratio of the alkali to the compound of formula I is 10:1 to 1:4; preferably 8:1 to 1:3, more preferably 5: 1 to 1:1.

13. The method according to claim 10, further comprising a step of purifying the compound of formula V, wherein the step of purifying the compound of formula V comprises adding a solvent to the crude product of the compound of formula V for layering, preferably the solvent used for layering is a mixture of one or a mixed solvent of two or more selected from the group consisting of alkane solvents, ether solvents and unsaturated aromatic hydrocarbon solvents, and an aqueous inorganic alkali solution;
further preferably,
the alkane solvent is a C₅-C₂₀ linear or branched or cyclic alkane;
the ether solvent is one or a mixture of two or more selected from the group consisting of tetrahydrofuran, methyl tert-butyl ether, diethyl ether, cyclopentyl methyl ether, isopropyl ether, anisole, ethylene glycol dimethyl ether, tetrahydropyran, and dioxane;
the unsaturated aromatic hydrocarbon solvent is selected from the group consisting of toluene, chlorobenzene, and xylene;
the inorganic alkali in the aqueous inorganic alkali solution of is one or a mixture of two or more selected from sodium hydroxide, potassium hydroxide, lithium hydroxide, sodium carbonate, potassium carbonate, and lithium carbonate, the mass fraction of the aqueous inorganic alkali solution is 1%-30%;
preferably, the step of purifying the compound of formula V further comprises: adjusting the pH of the organic phase to 6-7 with an acid after completion of layering, and after layering, concentrating the organic phase to dryness to obtain an oily compound of formula V;
wherein, preferably, the acid used is one of organic acids and inorganic acids, wherein, more preferably, the organic acid used is selected from the group consisting of formic acid, acetic acid, propionic acid, oxalic acid, maleic acid, citric acid, tartaric acid, methanesulfonic acid, and benzenesulfonic acid; wherein, further more preferably, the inorganic acid used is selected from the group consisting of hydrochloric acid, sulfuric acid, and phosphoric acid.

14. A crystal form A of cannabidiol, wherein:
the DSC spectrum data of the crystal form A are as follows: onset=65.46±1°C, peak=68.66±1°C;
the X-ray powder diffraction data of the crystal form A are as follows: there are X-ray diffraction peaks at 2θ of 5.097°±0.2°, 9.40°±0.2°, 9.71°±0.2°, 10.22°±0.2°, 11.79°±0.2°, 12.503°±0.2°, 13.147°±0.2°, 13.787°±0.2°, 15.086°±0.2°, 17.05°±0.2°, 17.40°±0.2°, 17.98°±0.2°, 19.00°±0.2°, 19.83°±0.2°, 20.891°±0.2°, 21.685°±0.2°22.17°±0.2°,22.60°±0.2°, 24.416°±0.2°, 29.091°±0.2°, and 31.133°±0.2°.

15. A method for preparing the crystal form A of cannabidiol, comprising: dissolving cannabidiol in an alkane solvent in amount of 0.2-10 times of the weight of the cannabidiol, cooling to a temperature of -50°C to 10°C, stirring or standing at the temperature, and then filtering or centrifuging the suspension to separate and obtain the crystal form A of cannabidiol, preferably, the alkane solvent is one or a mixture of two or more selected from the group consisting of C₄-C₂₀ linear or branched or cyclic alkanes.

16. The method according to claim 14, wherein the cannabidiol is prepared by the method of any one of claims 10-13.
